# EUROPEAN PATENT APPLICATION

(11) **EP 4 293 044 A1**
(43) Date of publication of application: **20.12.2023**
(21) Application number: 22739112.5
(22) Date of filing: 14.01.2022
(51) Int. Cl.: C07K 16/28, C12N 5/10, A61K 39/395, C12N 15/13, A61P 35/00

(54) **ANTI-TRPV6 MONOCLONAL ANTIBODY AND APPLICATION THEREOF**

(30) Priority: 15.01.2021 WO PCT/CN2021/072249; 11.01.2022 CN 202210029357
(71) Applicant: Coherent Biopharma (Suzhou), Limited, Industrial Park Suzhou Jiangsu 215123 (CN)
(72) Inventor: HUANG, Baohua Robert, Suzhou, Jiangsu 215123 (CN); TAN, Wei, Suzhou, Jiangsu 215123 (CN); WANG, Zhongbo, Suzhou, Jiangsu 215123 (CN); WANG, Wei, Suzhou, Jiangsu 215123 (CN)
(74) Representative: Lahrtz, Fritz
(86) International application number: PCT/CN2022/071976
(87) International publication number: WO 2022/152236

(57) **Abstract**

A segment of characteristic polypeptide antigen of a TRPV6 protein is selected to immunize mice and then screening is performed to obtain hybridoma cell lines 12CT 9.5.1, 16CT 18.1.1.2 and 16CT 4.1.1.2. The series of cells can secrete monoclonal antibodies against TRPV6 and can specifically recognize the TRPV6 protein and tumor tissue. The series of antibodies can measure the level of TRPV6 in cells in an artificial or automated manner by means of immunohistochemistry (IHC), enzyme-linked immunosorbent assay (ELISA), immunofluorescence (IF), chemiluminescence (CLA), immunoturbidimetric (TIA), or western blot, are thus used in a method for diagnosing tumor tissue, and relate to the field of biological detection.

## Description

### Technical Field

The present invention relates to a series of antibodies capable of recognizing human TRPV6 protein and a hybridoma cell line capable of secreting the antibodies. Specifically, the present invention provides a series of monoclonal antibodies specifically binding to TRPV6 molecules in the tumour cell membrane/cytoplasm, and the amino acid sequences of the heavy chain and light chain variable regions of the antibodies and the DNA sequences encoding the variable regions are determined. The antibodies or the antigen fragments thereof can be used to measure the level of TRPV6 in cells in an artificial or automated manner by means of immunohistochemistry (IHC), enzyme-linked immunosorbent assay (ELISA), immunofluorescence (IF), chemiluminescent immunoassay (CLIA), turbidimetric immunoassay (TIA), or western blot, are thus used in a method for diagnosing tumours, and relate to the field of biological detection.

### Background Art

Transient receptor potential cation channel subfamily V member 6 (TRPV6) is a highly selective calcium ion transmembrane transport channel that mediates the active transport of calcium ions from extracellular to intracellular. TRPV6 is expressed in normal human kidney, gastrointestinal tract, pancreas, breast, salivary gland, etc., and mainly expressed in intestinal epithelial cells. Since TRPV6 participates in the transport of calcium ions into a cell, when the number or function of TRPV6 channels changes, it can cause changes in the regulation of calcium ions, and further lead to structural or functional abnormalities of related tissues and organs.

Compared with normal tissues, the expression of TRPV6 is significantly increased in malignant tumours such as breast cancer, bile duct cancer, ovarian cancer, lung squamous cell carcinoma, and prostate cancer, and the abnormal expression thereof may be related to the formation and progression of the tumours. Prevarskaya's team found that the calcium ion uptake by human prostate cancer LNCap cells with high expression of TRPV6 is mediated by TRPV6, and after TRPV6 protein level was downregulated by siRNA treatment, the number of cells in S phase decreased and cell proliferation was inhibited. In addition, after human breast cancer MDA-MB-231 cells, prostate cancer PC-3 cells and ovarian cancer ES-2 cells were treated with lidocaine, the levels of TRPV6 mRNA and protein were down-regulated, cell proliferation was inhibited, and the rate at which calcium ions enter the cell was reduced, suggesting that the inhibition of cancer cells may be related to the reduction of calcium ions, and may be related to TRPV6 levels. It can be seen that TRPV6 is closely related to various tumours in the human body, which may be related to the regulation of intracellular and extracellular calcium ion concentrations by TRPV6.

Using TRPV6 as a tumour cell marker and the detection therefor are of great significance for pathological diagnosis and the use of a corresponding targeted drug. At present, there is no available anti-TRPV6 antibody on the market that can be used for diagnosis, and the antibody involved in the present patent can fill the gap.

### Summary of the Invention

One aspect of the present application provides an antibody or an antigen-binding fragment thereof specifically binding to TRPV6, wherein the antibody or the antigen-binding fragment thereof comprises three heavy chain complementarity determining regions HCDR1, HCDR2 and HCDR3 included in the following heavy chain variable regions: a heavy chain variable region shown in SEQ ID NO: 25; a heavy chain variable region shown in SEQ ID NO: 27; or a heavy chain variable region shown in SEQ ID NO: 29. In some embodiments, the antibody or the antigen-binding fragment thereof comprises three heavy chain complementarity determining regions HCDR1, HCDR2 and HCDR3 selected from the group of HCDR1 shown in SEQ ID NO: 7, HCDR2 shown in SEQ ID NO: 8, and HCDR3 shown in SEQ ID NO: 9; HCDR1 shown in SEQ ID NO: 13, HCDR2 shown in SEQ ID NO: 14, and HCDR3 shown in SEQ ID NO: 15; or HCDR1 shown in SEQ ID NO: 19, HCDR2 shown in SEQ ID NO: 20, and HCDR3 shown in SEQ ID NO: 21.

In some embodiments, the antibody or the antigen-binding fragment thereof further comprises three light chain complementarity determining regions LCDR1, LCDR2 and LCDR3 included in the following light chain variable region: a light chain variable region shown in SEQ ID NO: 26; a light chain variable region shown in SEQ ID NO: 28; or a light chain variable region shown in SEQ ID NO: 30. In some embodiments, the antibody or the antigen-binding fragment thereof further comprises three light chain complementarity determining regions LCDR1, LCDR2 and LCDR3 selected from the group of LCDR1 shown in SEQ ID NO: 10, LCDR2 shown in SEQ ID NO: 11 and LCDR3 shown in SEQ ID NO: 12; LCDR1 shown in SEQ ID NO: 16, LCDR2 shown in SEQ ID NO: 17 and LCDR3 shown in SEQ ID NO: 18; or LCDR1 shown in SEQ ID NO: 22, LCDR2 shown in SEQ ID NO: 23 and LCDR3 shown in SEQ ID NO: 24.

In some embodiments, the antibody or the antigen-binding fragment thereof comprises three heavy chain complementarity determining regions HCDR1, HCDR2 and HCDR3 and three light chain complementarity determining regions LCDR1, LCDR2 and LCDR3 selected from the group of HCDR1 shown in SEQ ID NO: 7, HCDR2 shown in SEQ ID NO: 8, HCDR3 shown in SEQ ID NO: 9, LCDR1 shown in SEQ ID NO: 10, LCDR2 shown in SEQ ID NO: 11 and LCDR3 shown in SEQ ID NO: 12; HCDR1 shown in SEQ ID NO: 13, HCDR2 shown in SEQ ID NO: 14, HCDR3 shown in SEQ ID NO: 15, LCDR1 shown in SEQ ID NO: 16, LCDR2 shown in SEQ ID NO: 17 and LCDR3 shown in SEQ ID NO: 18; or HCDR1 shown in SEQ ID NO: 19, HCDR2 shown in SEQ ID NO: 20, HCDR3 shown in SEQ ID NO: 21, LCDR1 shown in SEQ ID NO: 22, LCDR2 shown in SEQ ID NO: 23 and LCDR3 shown in SEQ ID NO: 24.

In some embodiments, the antibody or the antigen-binding fragment thereof comprises HCDR1 shown in SEQ ID NO: 7, HCDR2 shown in SEQ ID NO: 8, HCDR3 shown in SEQ ID NO: 9, LCDR1 shown in SEQ ID NO: 10, LCDR2 shown in SEQ ID NO: 11 and LCDR3 shown in SEQ ID NO: 12.

In some embodiments, the antibody or the antigen-binding fragment thereof comprises HCDR1 shown in SEQ ID NO: 13, HCDR2 shown in SEQ ID NO: 14, HCDR3 shown in SEQ ID NO: 15, LCDR1 shown in SEQ ID NO: 16, LCDR2 shown in SEQ ID NO: 17 and LCDR3 shown in SEQ ID NO: 18.

In some embodiments, the antibody or the antigen-binding fragment thereof comprises HCDR1 shown in SEQ ID NO: 19, HCDR2 shown in SEQ ID NO: 20, HCDR3 shown in SEQ ID NO: 21, LCDR1 shown in SEQ ID NO: 22, LCDR2 shown in SEQ ID NO: 23 and LCDR3 shown in SEQ ID NO: 24.

In some embodiments, the antibody or the antigen-binding fragment thereof comprises a heavy chain variable region V_{H} selected from the group of a heavy chain variable region shown in SEQ ID NO: 25; a heavy chain variable region shown in SEQ ID NO: 27; or a heavy chain variable region shown in SEQ ID NO: 29.

In some embodiments, the antibody or the antigen-binding fragment thereof further comprises a light chain variable region V_{L} selected from the group of a light chain variable region shown in SEQ ID NO: 26; a light chain variable region shown in SEQ ID NO: 28; or a light chain variable region shown in SEQ ID NO: 30.

In some embodiments, the antibody or the antigen-binding fragment thereof comprises a heavy chain variable region V_{H} and a light chain variable region V_{L} selected from the group of a heavy chain variable region shown in SEQ ID NO: 25 and a light chain variable region shown in SEQ ID NO: 26; a heavy chain variable region shown in SEQ ID NO: 27 and a light chain variable region shown in SEQ ID NO: 28; or a heavy chain variable region shown in SEQ ID NO: 29 and a light chain variable region shown in SEQ ID NO: 30.

In some embodiments, the antibody or the antigen-binding fragment thereof comprises a heavy chain variable region shown in SEQ ID NO: 25 and a light chain variable region shown in SEQ ID NO: 26.

In some embodiments, the antibody or the antigen-binding fragment thereof comprises a heavy chain variable region shown in SEQ ID NO: 27 and a light chain variable region shown in SEQ ID NO: 28.

In some embodiments, the antibody or the antigen-binding fragment thereof comprises a heavy chain variable region shown in SEQ ID NO: 29 and a light chain variable region shown in SEQ ID NO: 30.

In some embodiments, the antibody or the antigen-binding fragment thereof specifically recognizes or binds to a protein having an amino acid sequence shown in SEQ ID NO: 2.

In some embodiments, the Kd value of the antibody or the antigen-binding fragment thereof to the protein having the amino acid sequence shown in SEQ ID NO: 2 is 10⁻⁷-10⁻¹⁰ M. In some embodiments, the Kd value is 10⁻⁷-10⁻¹⁰ M, 10⁻⁸-10⁻¹⁰ M or 10⁻⁹-10⁻¹⁰ M. In some embodiments, the Kd value of the antibody or the antigen-binding fragment thereof to the protein having the amino acid sequence shown in SEQ ID NO: 2 is less than about 10⁻⁷M. In some embodiments, the Kd value is less than about 10⁻⁷ M, less than about 10⁻⁸ M, less than about 10⁻⁹ M or less than about 10⁻¹⁰ M. In some embodiments, the Kd value is detected by a surface plasmon resonance method.

In some embodiments, the antigen-binding fragment is selected from F(ab')₂, Fab', Fab, Fv, scFv, dsFv or dAb.

In some embodiments, the antibody or the antigen-binding fragment thereof further has a heavy chain constant region and/or a light chain constant region. In some embodiments, heavy chain constant region is derived from murine IgG1 or murine IgG2a. In some embodiments, heavy chain constant region is derived from human IgG1.

In some embodiments, the antibody or the antigen-binding fragment thereof further has a conjugated moiety. In some embodiments, the conjugated moiety is a therapeutic agent, a radioactive isotope, a detectable tag, a pharmacokinetically modified moiety or a purified moiety. The conjugated moiety is linked directly or via a linker.

In one aspect, the present application also provides an isolated nucleic acid encoding the antibody or the antigen-binding fragment thereof specifically binding to TRPV6 of the present application.

In some embodiments, the nucleic acid comprises one or two of a nucleic acid sequence shown in SEQ ID NO: 31 or a nucleic acid sequence having at least 90% sequence identity to SEQ ID NO: 31, and a nucleic acid sequence shown in SEQ ID NO: 32 or a nucleic acid sequence having at least 90% sequence identity to SEQ ID NO: 32. In some embodiments, the nucleic acid comprises one or two of a nucleic acid sequence shown in SEQ ID NO: 33 or a nucleic acid sequence having at least 90% sequence identity to SEQ ID NO: 33, and a nucleic acid sequence shown in SEQ ID NO: 34 or a nucleic acid sequence having at least 90% sequence identity to SEQ ID NO: 34. In some embodiments, the nucleic acid comprises one or two of a nucleic acid sequence shown in SEQ ID NO: 35 or a nucleic acid sequence having at least 90% sequence identity to SEQ ID NO: 35, and a nucleic acid sequence shown in SEQ ID NO: 6 or a nucleic acid sequence having at least 90% sequence identity to SEQ ID NO: 6.

In one aspect, the present application also provides a vector comprising the nucleic acid sequence of the present application.

In one aspect, the present application also provides a host cell comprising the vector of the present application.

In one aspect, the present application also provides a multispecific antibody comprising the antibody or the antigen-binding fragment thereof of the present application, and one or more antibodies or antigen-binding portions thereof specifically binding to other antigens.

In one aspect, the present application also provides an antibody conjugate comprising the antibody or the antigen-binding fragment thereof of the present application or the multispecific antibody of the present application.

In one aspect, the present application also provides a hybridoma cell strain secreting an anti-TRPV6 monoclonal antibody, wherein the hybridoma cell strain is 12CT9.5.1, 16CT4.1.1.2 and/or 16CT18.1.1.2 and deposited in China Center for Type Culture Collection, and the deposit number of 12CT9.5.1 is CCTCC NO: C202119, the deposit number of 16CT4.1.1.2 is CCTCC NO: C202124, and the deposit number of 16CT18.1.1.2 is CCTCC NO: C202125.

In one aspect, the present application also provides a pharmaceutical composition comprising the antibody or the antigen-binding fragment thereof of the present application, the isolated nucleic acid of the present application, the vector of the present application, the host cell of the present application, the multispecific antibody of the present application or the antibody conjugate of the present application, and a pharmaceutically acceptable carrier.

In one aspect, the present application also provides a reagent for analyzing and detecting TRPV6 in a sample from a subject, wherein the reagent comprises one or more of the antibodies or antigen-binding fragments thereof of the present application. In some embodiments, the reagent is used for analyzing and detecting TRPV6 in the sample from the subject.

In one aspect, the present application also provides a method for detecting TRPV6 in a sample from a subject, comprising: (1) contacting the sample with the antibody or the antigen-binding fragment of the present application; (2) optionally, removing unbound antibodies or antibody fragments; (3) detecting the antibody or antibody fragment binding to TRPV6 in the sample.

In some embodiments, the antibody or antigen-binding fragment of the present application is immobilized on a substrate in step (1) of the method for detecting TRPV6 in the sample from the subject. In some embodiments, the sample is immobilized on a substrate in step (1) of the method for detecting TRPV6 in the sample from the subject.

In some embodiments, the step (3) of the method for detecting TRPV6 in the sample from the subject further comprises using a second antibody, optionally, the second antibody can directly or indirectly bind to TRPV6 in the sample, optionally, the epitope of the second antibody is different from the epitope of the antibody or the antigen-binding fragment of the present application, optionally, the epitope of the second antibody is the same as the epitope of the antibody or the antigen-binding fragment of the present application, optionally, the binding of the epitope of the second antibody to TRPV6 is not affected by the antibody or antigen-binding fragment of the present application, and optionally, the binding of the epitope of the second antibody to TRPV6 is affected by the antibody or antigen-binding fragment of the present application.

In some embodiments, the step (3) of the method for detecting TRPV6 in the sample from the subject further comprises using a second antibody, optionally, the second antibody can directly or indirectly bind to the antibody or the antigen-binding fragment of the present application, and optionally, the second antibody can bind to the constant region portion of the antibody or the antigen-binding fragment of the present application.

In some embodiments, the second antibody is coupled to a detection molecule, and optionally, the detection molecule includes an enzyme, a fluorescent label and biotin. In some embodiments, the detection molecule is an enzyme, and optionally, the enzyme includes horseradish peroxidase, alkaline phosphatase or a derivative thereof. In some embodiments, the detection molecule is fluorescein, optionally, the fluorescein includes FITC, rhodamine, Texas Red, phycoerythrin or Dylight. In some embodiments, the detection molecule is biotin or a derivative thereof.

In some embodiments, the method for detecting TRPV6 in the sample from the subject further comprises quantifying the antibody binding to TRPV6 in the sample. In some embodiments, the method is immunohistochemistry (IHC), immunofluorescence (IF), enzyme-linked immunosorbent assay (ELISA), chemiluminescent immunoassay (CLIA), turbidimetric immunoassay (TIA), or western blot. In some embodiments, the method can be used to assess the proportion of the cells expressing TRPV6 on the cell surface in the sample.

In some embodiments, in the method for detecting TRPV6 in the sample from the subject, the subject is a human.

In one aspect, the present application also provides a method for determining disease formation or the risk of forming a disease in a subject, assessing the progression or prognosis of a disease in a subject, or predicting or monitoring the response of a subject being receiving a treatment for a disease to the treatment in the subject, wherein the method comprises detecting TRPV6 in the sample from the subject using the method for detecting TRPV6 in the sample from the subject of the present application.

In some embodiments, the method for determining disease formation or the risk of forming a disease in a subject, assessing the progression or prognosis of a disease in a subject, or predicting or monitoring the response of a subject being receiving a treatment for a disease to the treatment in the subject comprises assessing the proportion of cells expressing TRPV6 on the cell surface in the sample, and if the proportion exceeds a predetermined threshold, the subject is considered to have the disease or be more likely to benefit from the treatment, and wherein the threshold is 10%. In some embodiments, the treatment is a targeted drug therapy.

In some embodiments, the disease of the present application is cancer. In some embodiments, the cancer is breast cancer, ovarian cancer, endometrial cancer, bile duct cancer, gastric cancer, esophageal cancer, lung cancer, intestinal cancer, or pancreatic cancer.

In one aspect, the present application also provides a kit comprising the antibody or the antigen-binding fragment thereof of the present application, and optionally, further comprising a reagent for detecting the binding of the antibody or the antigen-binding fragment thereof to TRPV6.

In some embodiments, the kit of the present application is used for diagnosing disease formation or the risk of forming a disease in a subject, assessing the progression or prognosis of a disease in a subject, or predicting or monitoring the response of a subject being receiving a treatment for a disease to the treatment in the subject. In some embodiments, the kit of the present application is used for immunohistochemical pathological diagnosis. In some embodiments, the kit of the present application is used for immunohistochemical pathological diagnosis of tumour tissue. In some embodiments, the disease is cancer. In some embodiments, the cancer is breast cancer, ovarian cancer, endometrial cancer, bile duct cancer, gastric cancer, esophageal cancer, lung cancer, intestinal cancer, or pancreatic cancer.

In one aspect, the present application also provides a method for preparing an antibody specifically binding to TRPV6, comprising: (1) coupling the amino acid sequence shown in SEQ ID NO: 3 to a carrier protein to obtain TRPV6 antigen peptide; (2) immunizing a mouse with the TRPV6 antigen peptide obtained by step (1) as an immunogen; (3) performing cell fusion, and screening of the immunopeptide clones to obtain a positive hybridoma cell line that efficiently secretes an antibody specifically binding to TRPV6; (4) obtaining the antibody specifically binding to TRPV6.

In some embodiments, the carrier protein is keyhole limpet hemocyanin (KLH) protein.

In some embodiments, a Cys is added to the N-terminus of the amino acid sequence shown in SEQ ID NO: 3, which is then coupled to the carrier protein via a free sulfhydryl group.

In some embodiments, the hybridoma cell line of the present application is mouse hybridoma cell line 12CT 9.5.1, 16CT 18.1.1.2 or 16CT 4.1.1.2.

In one aspect, the present application also provides the use of the antibody or the antigen-binding fragment thereof of the present application, the isolated nucleic acid of the present application, the vector of the present application, the host cell of the present application, the multispecific antibody of the present application, the antibody conjugate of the present application and the pharmaceutical composition of the present application in a drug for treating and/or preventing and/or diagnosing diseases related to the TRPV6 level.

In one aspect, the present application also provides a method for treating, preventing or diagnosing diseases, wherein the method comprises administering the antibody or the antigen-binding fragment thereof of the present application, the multispecific antibody of the present application, the antibody conjugate of the present application and the pharmaceutical composition of the present application to a subject in need thereof.

### Brief Description of the Drawings

The drawings form a part of this description, and further illustrate certain aspects of the present disclosure. The present disclosure can be better understood by reference to one or more of the drawings in combination with the detailed description of specific embodiments presented in the present disclosure.

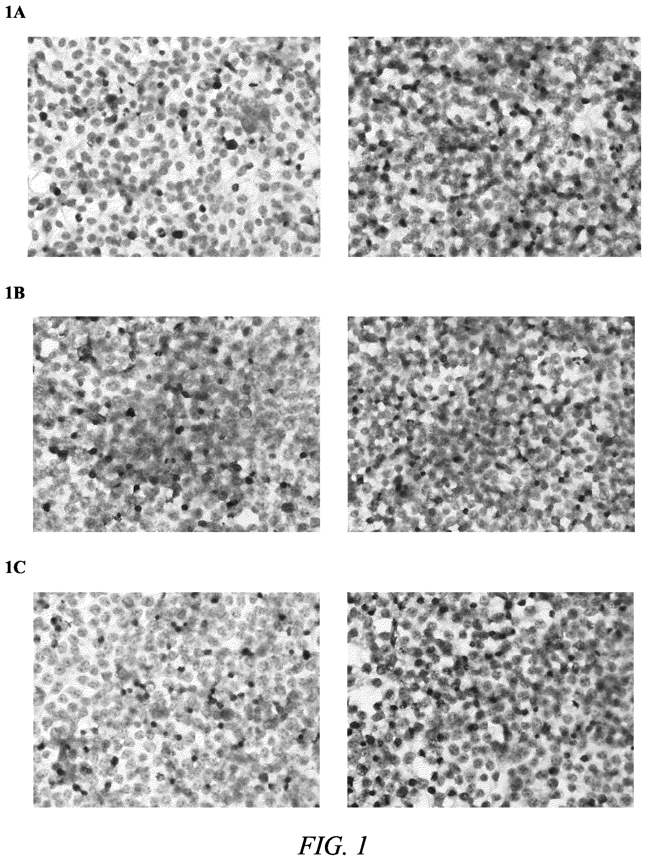

FIG. 1A shows that CB-Anti-0001 can specifically detect TRPV6 protein in TRPV6 plasmid-transfected 293T cells by immunocytochemistry (left panel), and can not detect TRPV6 protein in non-TRPV6 plasmid-transfected 293T cells (right panel). FIG. 1B shows that CB-Anti-0002 can specifically detect TRPV6 protein in TRPV6 plasmid-transfected 293T cells by immunocytochemistry (left panel), and can not detect TRPV6 protein in non-TRPV6 plasmid-transfected 293T cells (right panel). FIG. 1C shows that CB-Anti-0003 can specifically detect TRPV6 protein in TRPV6 plasmid-transfected 293T cells by immunocytochemistry (left panel), and can not detect TRPV6 protein in non-TRPV6 plasmid-transfected 293T cells (right panel).

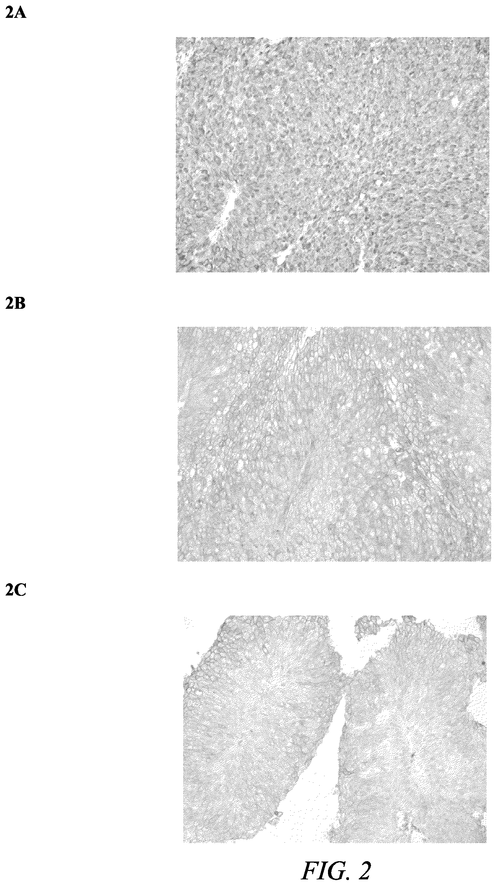

FIG. 2A shows the result of immunohistochemical detection of CB-Anti-0001 antibody. FIG. 2B shows the result of immunohistochemical detection of CB-Anti-0002 antibody. FIG. 2C shows the result of immunohistochemical detection of CB-Anti-0003 antibody.

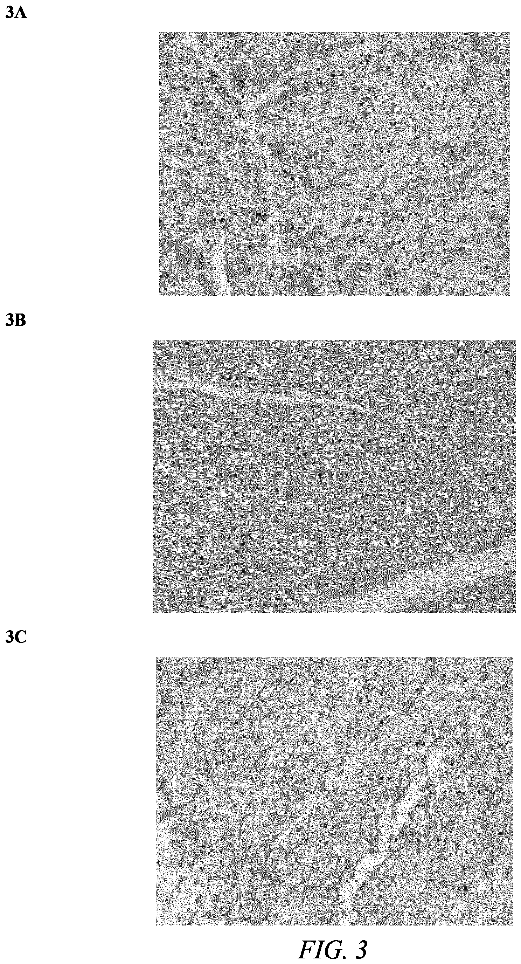

FIG. 3 shows the IHC comparison results of ACC-028 antibody, MABN839 antibody and CB-Anti-0001 antibody against human bladder transitional cell papilloma cells (RT4). FIG. 3A is the IHC result of ACC-028 antibody, FIG. 3B is the IHC result of MABN839 antibody, and FIG. 3C is the IHC result of CB-Anti-0001 antibody. It can be seen that the cell membrane in FIG. 3C has a clear and significant staining effect.

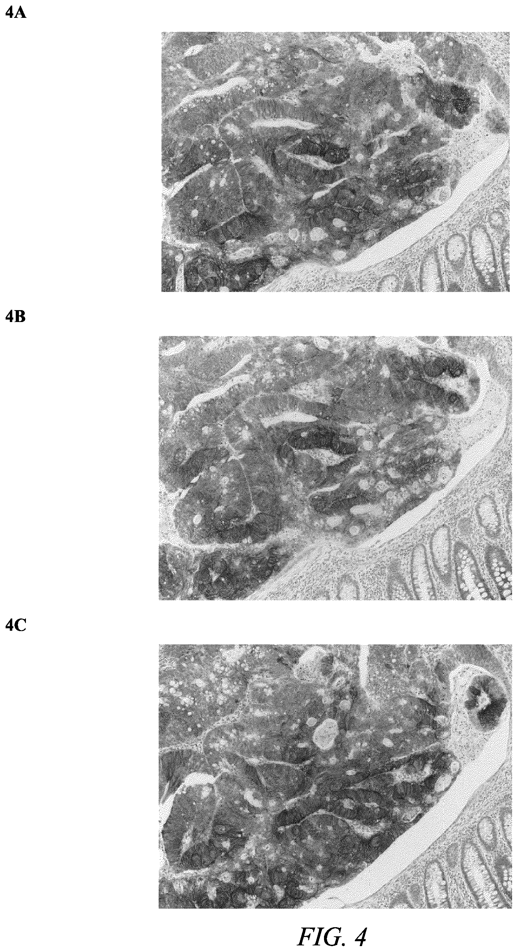

FIG. 4 shows that the tissue staining intensities and staining ratios of the antibodies stored under different conditions do not change significantly. FIG. 4A is the IHC result of the CB-Anti-0001 antibody stock solution stored at 4°C for 14 days, FIG. 4B is the IHC result of the CB-Anti-0001 antibody stock solution stored at 37°C for 14 days, and FIG. 4C is the IHC result of the CB-Anti-0001 antibody working solution stored at 37°C for 14 days.

### Detailed Description of the Invention

The following description is only intended to illustrate various embodiments of the present application. Therefore, the specific embodiments herein should not be construed as a limitation to the scope of the present application. Those skilled in the art can easily obtain various equivalents and modifications based on the principles of the present invention and the description herein, and it should be understood that such equivalent embodiments are included in the scope of the present invention. All references cited in the present application, including public publication, patent, and patent application, are incorporated into the present application by reference in their entirety.

Unless the context clearly indicates otherwise, the singular form "a", "an" and "the" as used herein includes plural reference.

Terms such as "include", "comprise", "contain", "containing" and "have" as used in the present application are inclusive or open-ended and do not exclude additional, unrecited element or method step. The term "consist of" as used in the present application is a closed expression.

The applicant has submitted the hybridoma cell strains (lines) 12CT9.5.1, 16CT4.1.1.2 and 16CT18.1.1.2 to the China Center for Type Culture Collection for preservation. The deposit number of 12CT9.5.1 is CCTCC NO: C202119, the deposit number of 16CT4.1.1.2 is CCTCC NO: C202124, and the deposit number of 16CT18.1.1.2 is CCTCC NO: C202125, and for the tri-hybridoma cell line, the deposit address is Wuhan University, the preservation time is 13 January 2021 and the preservation unit is China Center for Type Culture Collection.

One aspect of the present application discloses an antibody or an antigen-binding fragment thereof specifically binding to TRPV6, wherein the antibody or the antigen-binding fragment thereof comprises three heavy chain complementarity determining regions HCDR1, HCDR2 and HCDR3 included in the following heavy chain variable regions: a heavy chain variable region shown in SEQ ID NO: 25; a heavy chain variable region shown in SEQ ID NO: 27; or a heavy chain variable region shown in SEQ ID NO: 29. In some embodiments, the antibody or the antigen-binding fragment thereof comprises three heavy chain complementarity determining regions HCDR1, HCDR2 and HCDR3 selected from the group of HCDR1 shown in SEQ ID NO: 7, HCDR2 shown in SEQ ID NO: 8, and HCDR3 shown in SEQ ID NO: 9; HCDR1 shown in SEQ ID NO: 13, HCDR2 shown in SEQ ID NO: 14, and HCDR3 shown in SEQ ID NO: 15; or HCDR1 shown in SEQ ID NO: 19, HCDR2 shown in SEQ ID NO: 20, and HCDR3 shown in SEQ ID NO: 21. In some embodiments, the antibody or antigen-binding fragment thereof of the present application further comprises the antibody or the antigen-binding fragment thereof further comprises three light chain complementarity determining regions LCDR1, LCDR2 and LCDR3 included in the following light chain variable region: a light chain variable region shown in SEQ ID NO: 26; a light chain variable region shown in SEQ ID NO: 28; or a light chain variable region shown in SEQ ID NO: 30. In some embodiments, the antibody or the antigen-binding fragment thereof of the present application further comprises three light chain complementarity determining regions LCDR1, LCDR2 and LCDR3 selected from the group of LCDR1 shown in SEQ ID NO: 10, LCDR2 shown in SEQ ID NO: 11 and LCDR3 shown in SEQ ID NO: 12; LCDR1 shown in SEQ ID NO: 16, LCDR2 shown in SEQ ID NO: 17 and LCDR3 shown in SEQ ID NO: 18; or LCDR1 shown in SEQ ID NO: 22, LCDR2 shown in SEQ ID NO: 23 and LCDR3 shown in SEQ ID NO: 24.

In some embodiments, the antibody or the antigen-binding fragment thereof of the present application comprises HCDR1 shown in SEQ ID NO: 7, HCDR2 shown in SEQ ID NO: 8, HCDR3 shown in SEQ ID NO: 9, LCDR1 shown in SEQ ID NO: 10, LCDR2 shown in SEQ ID NO: 11 and LCDR3 shown in SEQ ID NO: 12. In some embodiments, the antibody or the antigen-binding fragment thereof of the present application comprises HCDR1 shown in SEQ ID NO: 13, HCDR2 shown in SEQ ID NO: 14, HCDR3 shown in SEQ ID NO: 15, LCDR1 shown in SEQ ID NO: 16, LCDR2 shown in SEQ ID NO: 17 and LCDR3 shown in SEQ ID NO: 18. In some embodiments, the antibody or the antigen-binding fragment thereof of the present application comprises HCDR1 shown in SEQ ID NO: 19, HCDR2 shown in SEQ ID NO: 20, HCDR3 shown in SEQ ID NO: 21, LCDR1 shown in SEQ ID NO: 22, LCDR2 shown in SEQ ID NO: 23 and LCDR3 shown in SEQ ID NO: 24.

In some embodiments, the antibody or the antigen-binding fragment thereof of the present application comprises a heavy chain variable region V_{H} selected from the group of a heavy chain variable region shown in SEQ ID NO: 25; a heavy chain variable region shown in SEQ ID NO: 27; or a heavy chain variable region shown in SEQ ID NO: 29. In some embodiments, the antibody or the antigen-binding fragment thereof further comprises a light chain variable region V_{L} selected from the group of a light chain variable region shown in SEQ ID NO: 26; a light chain variable region shown in SEQ ID NO: 28; or a light chain variable region shown in SEQ ID NO: 30.

In some embodiments, the antibody or the antigen-binding fragment thereof of the present application comprises a heavy chain variable region shown in SEQ ID NO: 25 and a light chain variable region shown in SEQ ID NO: 26. In some embodiments, the antibody or the antigen-binding fragment thereof of the present application comprises a heavy chain variable region shown in SEQ ID NO: 27 and a light chain variable region shown in SEQ ID NO: 28. In some embodiments, the antibody or the antigen-binding fragment thereof of the present application comprises a heavy chain variable region shown in SEQ ID NO: 29 and a light chain variable region shown in SEQ ID NO: 30.

The term "antibody" as used in the present application includes any immunoglobulin, monoclonal antibody, multivalent antibody, multispecific antibody or bispecific (bivalent) antibody. A natural intact antibody comprises two heavy chains and two light chains which are bound to each other by disulfide bonds. Each heavy chain of an antibody consists of a variable region (V_{H}) and first, second and third constant regions (respectively C_{H1}, C_{H2} and C_{H3}), and each light chain of an antibody consists of a variable region (V_{L}) and a constant region (C_{L}). The variable regions of the light and heavy chains are responsible for antigen binding. The variable region in each chain is roughly subdivided into 3 hypervariable regions, i.e., complementarity determining regions (CDRs) (the CDRs of the light chain include LCDR1, LCDR2 and LCDR3, and the CDRs of the heavy chain include HCDR1, HCDR2 and HCDR3).

The term "antigen-binding fragment" of an antibody, "antigen-binding portion" of an antibody, etc., as used in the present application includes any naturally occurring, enzymatically obtainable, synthetic or genetically engineered polypeptide that specifically binds to an antigen to form a complex. The antigen-binding fragment of the antibody can, for example, be derived from an intact antibody molecule by using any suitable standard technique, such as proteolytic digestion or recombinant genetic engineering technique involving the manipulation and expression of DNA encoding the variable domain and optionally constant domain of the antibody. DNA can be sequenced and manipulated by a chemical method or using a molecular biology technique, thereby arranging one or more variable and/or constant domains into a suitable configuration, or introducing codon, generating a cysteine residue, performing modification, addition or deletion of amino acid, etc. Non-limiting examples of the antigen-binding fragment include: (i) Fab fragment; (ii) F(ab')2 fragment; (iii) Fd fragment; (iv) Fv fragment; (v) single chain Fv (scFv) molecule; (vi) dAb fragment; and (vii) a minimal recognition unit that mimics the amino acid residue composition of an antibody hypervariable region (e.g., an isolated complementarity determining region (CDR), such as CDR3 peptide) or FR3-CDR3-FR4 peptide. Other engineered molecules such as a domain-specific antibody, single-domain antibody, domain-deleted antibody, chimeric antibody, CDR-grafted antibody, diabody, triabody, tetrabody, minibody, nanobody (e.g., monovalent nanobody, divalent nanobody, etc.), small modular immuno pharmaceuticals (SMIPs) and shark variant IgNAR domain are also included within the expression "antigen-binding fragment" used herein.

CDR boundaries for the antibodies and antigen-binding fragments disclosed in the present application can be named or identified by Kabat, Chothia or Al-Lazikani nomenclature. (Al-Lazikani, B., Chothia, C., Lesk, A. M., J. Mol. Biol., 273(4), 927 (1997); Chothia, C et al., J Mol Biol. Dec 5;186(3):651-63 (1985); Chothia, C. and Lesk, A.M., J.Mol.Biol., 196, 901 (1987); Chothia, C et al., Nature. Dec 21-28;31(6252):877-83 (1989); Kabat E.A. et al., National Institutes of Health, Bethesda, Md. (1991)). In some embodiments, the CDR boundaries of the antibody are determined according to the Kabat database. The three CDRs are interposed between flanking continuous portions known as framework regions (FR, wherein heavy chain FR includes HFR1, HFR2, HFR3 and HFR4, and light chain FR includes LFR1, LFR2, LFR3 and LFR4), which are more highly conserved than the CDRs and form a scaffold to support the hypervariable loops. Therefore, V_{H} and V_{L} each comprise 3 CDRs and 4 FRs arranged in the following order (from human amino acid residue N-terminus to C-terminus): FR1, CDR1, FR2, CDR2, FR3, CDR3, FR4. The constant regions of the heavy and light chains are not involved in antigen binding but exhibit various effector functions. Heavy chains of mammals are classified into α, δ, ε, γ and µ, and light chains of mammals are classified into λ or κ. Antibodies can be classified into the following 5 categories according to the amino acid sequence of the heavy chain constant region thereof and whether there are α, δ, ε, γ and µ respectively: IgA, IgD, IgE, IgG and IgM. Several major antibody subclasses are such as IgG1 (γ1 heavy chain), IgG2 (γ2 heavy chain), IgG3 (γ3 heavy chain), IgG4 (γ4 heavy chain), IgA1 (α1 heavy chain) or IgA2 (α2 heavy chain).

In some embodiments, the antibody or the antigen-binding fragment thereof of the present application specifically recognizes or binds to a protein having an amino acid sequence shown in SEQ ID NO: 2.
SEQ ID NO: 2:

In some embodiments, the Kd value of the antibody or the antigen-binding fragment thereof of the present application to the protein having the amino acid sequence shown in SEQ ID NO: 2 is 10⁻⁷-10⁻¹⁰ M. In some embodiments, the Kd value is 10⁻⁷-10⁻¹⁰ M, 10⁻⁸-10⁻¹⁰ M or 10⁻⁹-10⁻¹⁰ M. In some embodiments, the Kd value of the antibody or the antigen-binding fragment thereof to the protein having the amino acid sequence shown in SEQ ID NO: 2 is less than about 10⁻⁷M. In some embodiments, the Kd value is less than about 10⁻⁷ M, less than about 10⁻⁸ M, less than about 10⁻⁹ M or less than about 10⁻¹⁰ M. In some embodiments, the Kd value is detected by a surface plasmon resonance method.

In some embodiments, the antigen-binding fragment of the present application is selected from F(ab')₂, Fab', Fab, Fv, scFv, dsFv or dAb. In some embodiments, the antibody or the antigen-binding fragment thereof of the present application further has a heavy chain constant region and/or a light chain constant region. In some embodiments, heavy chain constant region is derived from murine IgG1 or murine IgG2a. In some embodiments, heavy chain constant region is derived from human IgG1.

In some embodiments, the antibody or the antigen-binding fragment thereof of the present application further has a conjugated moiety. In some embodiments, the conjugated moiety is a therapeutic agent, a radioactive isotope, a detectable tag, a pharmacokinetically modified moiety or a purified moiety. The conjugated moiety is linked directly or via a linker.

The term "therapeutic agent" as used in the present application can be any pharmaceutical compound used for treating or preventing a disease in a subject, including a small molecule with a molecular weight of less than 500, nucleotide (e.g., DNA, plasmid DNA, RNA, siRNA, antisense oligonucleotide, aptamer, etc.), short peptide, and protein (e.g., enzyme).

In some embodiments, the radioactive isotope can be selected from the group consisting of: ³H, ¹¹C, ¹⁴C, ¹⁸F, ³²P, ³³P, ³⁵S, ⁴⁵Ti, ⁴⁷Sc, ⁵²Fe, ⁵⁹Fe, ⁶²Cu, ⁶⁴Cu, ⁶⁷Cu, ⁶⁷Ga, ⁶⁸Ga, ⁷⁵Sc, ⁷⁷As, ⁸⁶Y, ⁸⁹Sr, ⁸⁹Zr, ⁹⁰Y, ⁹⁰Nb, ⁹⁴Tc, ⁹⁹Tc, ⁹⁹Mo, ¹⁰⁵Pd, ¹⁰⁵Rh, ¹¹¹Ag, ¹¹¹In, ¹²³I, ¹²⁴I, ¹²⁵I, ¹³¹I, ¹³³Xe, ¹⁴²Pr, ¹⁴³Pr, ¹⁴⁹Pm, ¹⁵³Sm, ¹⁵⁴Gd, ¹⁵⁵Gd, ¹⁵⁶Gd, ¹⁵⁷Gd, ¹⁵⁸Gd, ¹⁶¹Tb, ¹⁶⁶Dy, ¹⁶⁹Er, ¹⁷⁵Lu, ¹⁷⁷Lu, ¹⁸⁶Re, ¹⁸⁸Re, ¹⁸⁹Re, ¹⁹⁴Ir, ¹⁹⁸Au, ¹⁹⁹Au, ²¹¹At, ²¹¹Pb, ²¹²Bi, ²¹²Pb, ²¹³Bi, ²²³Ra and ²²⁵Ac.

The term "detectable tag" as used in the present application can be any detection molecule that is directly or indirectly coupled to the antibody of the present invention and is suitable for or helpful for detecting the level of TRPV6. In some embodiments, the detection molecule includes an enzyme, a fluorescent label and biotin. The enzyme includes peroxidase (e.g., horseradish peroxidase), alkaline phosphatase, β-galactosidase, glucoamylase, sugar oxidase, urease, heterocyclic oxidase (e.g., xanthine oxidase), malate dehydrogenase or a derivative thereof. The fluorescent label can be fluorescein, optionally, the fluorescein includes fluorescein isothiocyanate (FITC), rhodamine, Texas Red, phycoerythrin, green fluorescent protein (GFP), Dylight, Cy3 or Cy5.

In another aspect, the present application also discloses an isolated nucleic acid encoding the antibody or the antigen-binding fragment thereof specifically binding to TRPV6 of the present application. In some embodiments, the nucleic acid comprises one or two of a nucleic acid sequence shown in SEQ ID NO: 31 or a nucleic acid sequence having at least 90% sequence identity to SEQ ID NO: 31, and a nucleic acid sequence shown in SEQ ID NO: 32 or a nucleic acid sequence having at least 90% sequence identity to SEQ ID NO: 32. In some embodiments, the nucleic acid comprises one or two of a nucleic acid sequence shown in SEQ ID NO: 33 or a nucleic acid sequence having at least 90% sequence identity to SEQ ID NO: 33, and a nucleic acid sequence shown in SEQ ID NO: 34 or a nucleic acid sequence having at least 90% sequence identity to SEQ ID NO: 34. In some embodiments, the nucleic acid comprises one or two of a nucleic acid sequence shown in SEQ ID NO: 35 or a nucleic acid sequence having at least 90% sequence identity to SEQ ID NO: 35, and a nucleic acid sequence shown in SEQ ID NO: 6 or a nucleic acid sequence having at least 90% sequence identity to SEQ ID NO: 6.

In another aspect, the present application also discloses a vector comprising the nucleic acid sequence of the present application.

In another aspect, the present application also discloses a host cell comprising the vector of the present application.

In another aspect, the present application also discloses a multispecific antibody comprising the antibody or the antigen-binding fragment thereof of the present application, and one or more antibodies or antigen-binding portions thereof specifically binding to other antigens.

In another aspect, the present application also discloses an antibody conjugate comprising the antibody or the antigen-binding fragment thereof of the present application or the multispecific antibody of the present application.

In another aspect, the present application also discloses a hybridoma cell strain secreting an anti-TRPV6 monoclonal antibody, wherein the hybridoma cell strain is 12CT9.5.1, 16CT4.1.1.2 and/or 16CT18.1.1.2 and deposited in China Center for Type Culture Collection, and the deposit number of 12CT9.5.1 is CCTCC NO: C202119, the deposit number of 16CT4.1.1.2 is CCTCC NO: C202124, and the deposit number of 16CT18.1.1.2 is CCTCC NO: C202125.

In another aspect, the present application also discloses a pharmaceutical composition comprising the antibody or the antigen-binding fragment thereof of the present application, the isolated nucleic acid of the present application, the vector of the present application, the host cell of the present application, the multispecific antibody of the present application or the antibody conjugate of the present application, and a pharmaceutically acceptable carrier.

The term "pharmaceutically acceptable" as used in the present application refers to, within the scope of sound medical judgment, being suitable for contact with cells of human beings and other animals without undue toxicity, irritation, allergic response, etc., and being commensurate with a reasonable benefit/risk ratio.

The term "pharmaceutically acceptable carrier" as used in the present application refers to a pharmaceutically acceptable solvent, suspension or any other pharmacologically inert vehicle for delivering the antibody or the antigen-binding fragment thereof, isolated nucleic acid, vector, host cell, multispecific antibody or antibody conjugate provided in the present application to a subject, without interfering with the structure, morphology and property of the antibody or the antigen-binding fragment thereof, isolated nucleic acid, vector, host cell, multispecific antibody or antibody conjugate. Some of such carriers enable the antibody or the antigen-binding fragment thereof, isolated nucleic acid, vector, host cell, multispecific antibody or antibody conjugate to be formulated as, for example, tablets, pills, capsules, liquids, gels, syrups, slurries, suspensions and pastilles, for oral ingestion by a subject. Some of such carriers enable the antibody or the antigen-binding fragment thereof, isolated nucleic acid, vector, host cell, multispecific antibody or antibody conjugate to be formulated as preparations for injection, infusion or local administration.

The pharmaceutically acceptable carriers for use in the pharmaceutical composition provided in the present application may include, but are not limited to, for example, pharmaceutically acceptable liquids, gels, or solid carriers, aqueous vehicles (such as sodium chloride injection, Ringer's injection, isotonic dextrose injection, sterile water injection, or dextrose and lactated Ringer's injection), nonaqueous vehicles (such as fixed oils derived from vegetables, cottonseed oil, corn oil, sesame oil, or peanut oil), antimicrobial agents, isotonic agents (such as sodium chloride or dextrose), buffers (such as phosphate or citrate buffers), antioxidants (such as sodium bisulfate), anesthetics (such as procaine hydrochloride), suspensions/dispersions (such as sodium carboxymethylcellulose, hydroxypropyl methylcellulose, or polyvinylpyrrolidone), chelating agents (such as EDTA (ethylenediamine tetraacetic acid) or EGTA (ethylene glycol tetraacetic acid)), emulsifying agents (such as polysorbate 80 (Tween-80)), diluents, adjuvants, excipients or non-toxic auxiliary substances, other components well known in the art, or various combinations thereof. Suitable components may include, for example, fillers, binders, buffers, preservatives, lubricants, flavoring agents, thickening agents, colouring agents, or emulsifying agents.

In some embodiments, the pharmaceutical composition is an injection preparation. The injection preparations include sterile water solutions or dispersions, suspensions or emulsions. In all cases, the injection preparations should be sterile and be a fluid for easy injection. It should be stable under the conditions of manufacture and storage and must be preserved against the contaminating action of microorganisms such as bacteria and fungi. The carriers can be solvents or dispersion mediums containing, for example, water, ethanol, polyol (for example, glycerol, propylene glycol, and liquid polyethylene glycol), and suitable mixtures thereof and/or vegetable oils. The injection preparations should maintain appropriate fluidity. The appropriate fluidity can be maintained, for example, by the use of coatings such as lecithin, by the use of surfactants, and the like. Prevention of the action of microorganisms can be achieved by various antibacterial and antifungal agents, for example, parabens, chlorobutanol, phenol, sorbic acid, thimerosal, and the like.

In some embodiments, the pharmaceutical composition is an oral preparation. The oral preparations include, but are not limited to, capsules, cachets, pills, tablets, lozenges (using a flavored basis, usually sucrose and acacia or tragacanth), powders, granules, or as solutions or suspensions in aqueous or non-aqueous liquids, or as oil-in-water or water-in-oil liquid emulsions, or as elixirs or syrups, or as pastilles (using an insert base, such as gelatin and glycerin, or sucrose and acacia) and/or as mouth washes.

In solid dosage forms for oral administration (e.g., capsules, tablets, pills, dragees, pulvis, granules, etc.), the antibody or the antigen-binding fragment thereof, isolated nucleic acid, vector, host cell, multispecific antibody or antibody conjugate is mixed with one or more pharmaceutically acceptable carriers, such as sodium citrate or dicalcium phosphate, and/or any of the followings: (1) fillers or extenders, such as starches, lactose, sucrose, glucose, mannitol, and/or silicic acid; (2) binders, such as carboxymethylcellulose, alginates, gelatins, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as acetyl alcohol and glycerol monostearate; (8) absorbents, such as kaolin and bentonite clay; (9) lubricants, such as talc, calcium stearate, magnesium stearate, solid polyethylene glycol, sodium lauryl sulfate, and mixtures thereof; and (10) colouring agents.

In liquid dosage forms for oral administration, the antibody or the antigen-binding fragment thereof, isolated nucleic acid, vector, host cell, multispecific antibody or antibody conjugate is mixed with any of the followings: pharmaceutically acceptable emulsions, microemulsions, solutions, suspensions, syrups and elixirs. In addition to the antibody or the antigen-binding fragment thereof, isolated nucleic acid, vector, host cell, multispecific antibody or antibody conjugate, the liquid dosage forms may contain inert diluents commonly used in the art, such as, water or other solvents, solubilizing agents and emulsifying agents, such as ethyl alcohol, isopropyl alcohol, ethyl carbonate, ethyl acetate, benzyl alcohol, benzyl benzoate, isopropanol, 1,3-butylene glycol, oils (in particular, cottonseed oil, peanut oil, corn oil, olive oil, castor oil and sesame oil), glycerol, tetrahydrofurfuryl alcohol, polyethylene glycol and fatty acid esters of sorbitan, and mixtures thereof. Besides inert diluents, an oral composition can also include adjuvants such as wetting agents, emulsifying agents and suspensions, sweetening agents, flavoring agents, colouring agents, perfuming agents and preservatives.

In some embodiments, the pharmaceutical composition is a mouth spray preparation or a nasal spray preparation. The spray preparations include, but are not limited to, aqueous aerosols, nonaqueous suspensions, lipidosome preparations or solid granular preparations. Aqueous aerosols are prepared by mixing aqueous solutions or suspensions of agents with conventional pharmaceutically acceptable carriers and stabilizers. The carriers and stabilizers vary according to the requirements of specific pharmaceutical ingredients, but in general, they include nonionic surfactants (Tween or polyethylene glycol), oleic acid, lecithin, amino acids such as glycine, buffer solution, salts, sugar or sugar alcohol. Aerosols are generally prepared from isotonic solutions, and can be delivered by sprayers.

In some embodiments, the pharmaceutical composition can be used by mixing with one or more other drugs. In some embodiments, the pharmaceutical composition comprises at least one other drug. In some embodiments, the other drugs are antineoplastic drugs, cardiovascular drugs, anti-inflammatory drugs, antiviral drugs, digestive system drugs, nervous system drugs, respiratory system drugs, immune system drugs, dermatologic drugs, metabolic drugs, etc.

In some embodiments, the pharmaceutical compositions can be administered to a subject in need thereof by appropriate routes, including without limitation, oral, injection (such as intravenous, intramuscular, subcutaneous, intracutaneous, intracardiac, intrathecal, intrapleural and intraperitoneal injection), mucosal (such as nasal and intraoral administration), sublingual, rectal, percutaneous, intraocular, and pulmonary administration. In some embodiments, the pharmaceutical composition can be administered intravenously, subcutaneously, orally, intramuscularly or intraventricularly.

In another aspect, the present application also discloses a reagent for analyzing and detecting TRPV6 in a sample from a subject, wherein the reagent comprises one or more the antibodies or antigen-binding fragments thereof of the present application. In some embodiments, the reagent is used for analyzing and detecting TRPV6 in the sample from the subject.

In another aspect, the present application also discloses a method for detecting TRPV6 in a sample from a subject, comprising: (1) contacting the sample with the antibody or the antigen-binding fragment of the present application; (2) optionally, removing unbound antibodies or antibody fragments; (3) detecting the antibody or antibody fragment binding to TRPV6 in the sample.

The term "detection" as used in the present application can be performed by determining and measuring the presence or absence of a molecule (e.g., a polypeptide, protein or nucleic acid molecule), or by determining and measuring an interaction (e.g., binding, agonizing or antagonism, etc.) between molecules, such as protein-protein interaction (e.g., the interaction between an antibody and an antigen), protein-nucleic acid interaction, or nucleic acid-nucleic acid interaction. In some embodiments, "detection" and "measurement" include quantitative detection and measurement. In some embodiments, detection is performed by determining and measuring the signal of a detection molecule coupling directly or indirectly to the antibody. Detection can be performed by methods commonly known to those skilled in the art. In some embodiments, detection is performed by using immunohistochemistry (IHC), enzyme-linked immunosorbent assay (ELISA), immunofluorescence (IF), chemiluminescent immunoassay (CLIA), turbidimetric immunoassay (TIA), or western blot.

In some embodiments, the antibody or antigen-binding fragment of the present application is immobilized on a substrate in step (1) of the method for detecting TRPV6 in the sample from the subject. In some embodiments, the sample is immobilized on a substrate in step (1) of the method for detecting TRPV6 in the sample from the subject. In some embodiments, the sample is a cell or tissue. The sample includes, but is not limited to, a cell centrifugal preparation, a cytological smear, a core biopsy sample, a fine needle aspiration sample, and/or tissue slice (e.g., cryostat tissue slice, paraffin-embedded tissue slice). In some embodiments, the sample comprises a live cell. In some embodiments, the sample comprises a tumour cell. In some embodiments, the immobilization is performed using a chemical immobilizing agent. The histological sample can be prepared using a cross-linked immobilizing agent, such as aldehydes, including, for example, formaldehyde, paraformaldehyde, and glutaraldehyde. The tissue sample can be immobilized with formaldehyde and embedded in paraffin. The sample can also be immobilized with paraformaldehyde, embedded in a temperature-sensitive cryogenic material, and quick-frozen with liquid nitrogen. Other types of chemical immobilizing agents further include an oxidizing agent and an alcoholic immobilizing agent. The sample can also be immobilized by a physical method.

In some embodiments, the step (3) of the method for detecting TRPV6 in the sample from the subject further comprises using a second antibody, optionally, the second antibody can directly or indirectly bind to TRPV6 in the sample, optionally, the epitope of the second antibody is different from the epitope of the antibody or the antigen-binding fragment of the present application, optionally, the epitope of the second antibody is the same as the epitope of the antibody or the antigen-binding fragment of the present application, optionally, the binding of the epitope of the second antibody to TRPV6 is not affected by the antibody or antigen-binding fragment of the present application, and optionally, the binding of the epitope of the second antibody to TRPV6 is affected by the antibody or antigen-binding fragment of the present application.

The term "epitope" as used in the present application refers to an antigenic determinant that interacts with a specific antigen-binding site (called a paratope) in the variable region of an antibody molecule. A single antigen can have more than one epitope. Therefore, different antibodies can bind to different regions on one antigen and can have different biological effects. An epitope can be a conformational epitope or a linear epitope. The conformational epitope is produced by the spatial juxtaposition of amino acids from different segments of a linear polypeptide chain. The linear epitope is an epitope produced from adjacent amino acid residues in a polypeptide chain. In some cases, an epitope may comprise a sugar, phosphoryl or sulfonyl moiety on an antigen.

In some embodiments, the step (3) of the method for detecting TRPV6 in the sample from the subject further comprises using a second antibody, optionally, the second antibody can directly or indirectly bind to the antibody or the antigen-binding fragment of the present application, and optionally, the second antibody can bind to the constant region portion of the antibody or the antigen-binding fragment of the present application. The "second antibody" as used herein is intended to detect the anti-TRPV6 antibody in a better way, and the second antibody can generally be obtained commercially. In some embodiments, the second antibody of the present application is coupled to a detection molecule, and optionally, the detection molecule includes an enzyme, a fluorescent label and biotin. In some embodiments, the detection molecule is an enzyme, and optionally, the enzyme includes peroxidase (e.g., horseradish peroxidase), alkaline phosphatase, β-galactosidase, glucoamylase, sugar oxidase, urease, heterocyclic oxidase (e.g., xanthine oxidase), malate dehydrogenase or a derivative thereof. In some embodiments, the detection molecule is fluorescein, optionally, the fluorescein includes FITC, rhodamine, Texas Red, phycoerythrin, GFP, Cy3, Cy5 or Dylight. In some embodiments, the detection molecule is biotin or a derivative thereof.

In some embodiments, the method for detecting TRPV6 in the sample from the subject further comprises quantifying the antibody binding to TRPV6 in the sample. In some embodiments, the method is immunohistochemistry (IHC), enzyme-linked immunosorbent assay (ELISA), immunofluorescence (IF), chemiluminescent immunoassay (CLIA), turbidimetric immunoassay (TIA), or western blot. In some embodiments, the method can be used to assess the proportion of the cells expressing TRPV6 on the cell surface in the sample.

In some embodiments, in the method for detecting TRPV6 in the sample from the subject, the subject is a human.

In another aspect, the present application also discloses a method for determining disease formation or the risk of forming a disease in a subject, assessing the progression or prognosis of a disease in a subject, or predicting or monitoring the response of a subject being receiving a treatment for a disease to the treatment in the subject, wherein the method comprises detecting TRPV6 in the sample from the subject using the method for detecting TRPV6 in the sample from the subject of the present application.

In some embodiments, the method for determining disease formation or the risk of forming a disease in a subject, assessing the progression or prognosis of a disease in a subject, or predicting or monitoring the response of a subject being receiving a treatment for a disease to the treatment in the subject comprises assessing the proportion of cells expressing TRPV6 on the cell surface in the sample, and if the proportion exceeds a predetermined threshold, the subject is considered to have the disease or be more likely to benefit from the treatment, and wherein the threshold is 10%. In some embodiments, the treatment is a targeted drug therapy.

The term "benefit" or "beneficial" as used in the present application refers to any desired effect, including but not limited to: (1) inhibiting (such as reducing, slowing down or completely stopping) the development of the disease to some extent, including slowing down and completely stopping; (2) reducing the number of disease onsets and/or symptoms; (3) reducing the size of the lesion; (4) inhibiting the infiltration of diseased cells into an adjacent surrounding organ and/or tissue; (5) inhibiting the spread of a disease; (6) alleviating one or more symptoms related to the disease to a certain extent; (7) increasing the disease-free time after treatment; (8) reducing autoimmune response, which may but not necessarily lead to regression or ablation of disease lesions, such as progression-free survival; (9) increasing the overall survival rate; (10) higher response rate; and/or (11) reducing the mortality at a given time point after treatment.

In some embodiments, predicting or monitoring the response of the subject to a treatment involves assessing whether the tumour of the subject is in remission or progression. In some embodiments, predicting or monitoring the response of the subject to a treatment may involve assessing whether the subject is on the mend after receiving a treatment (e.g., a treatment with a particular drug) and/or the possibility of improvement. The predicting or monitoring method of the present application can be used clinically to make treatment decisions. The predicting or monitoring method of the present application is a valuable tool in terms of assessing the possibility whether a particular subject will survive for a long-term after a treatment regimen (e.g., a given treatment regimen, including, for example, administration of a given therapeutic drug or combination, surgical intervention, etc.).

In some embodiments, the disease of the present application is cancer. In some embodiments, the cancer is breast cancer, ovarian cancer, endometrial cancer, bile duct cancer, gastric cancer, esophageal cancer, lung cancer, intestinal cancer, or pancreatic cancer. In some embodiments, the cancer is HER2⁺ breast cancer, triple negative breast cancer, or colorectal cancer.

In another aspect, the present application also discloses a kit comprising the antibody or the antigen-binding fragment thereof of the present application, and optionally, further comprising a reagent for detecting the binding of the antibody or the antigen-binding fragment thereof to TRPV6.

In some embodiments, the kit of the present application is used for diagnosing disease formation or the risk of forming a disease in a subject, assessing the progression or prognosis of a disease in a subject, or predicting or monitoring the response of a subject being receiving a treatment for a disease to the treatment in the subject. In some embodiments, the kit of the present application is used for immunohistochemical pathological diagnosis. In some embodiments, the kit of the present application is used for immunohistochemical pathological diagnosis of tumour tissue. In some embodiments, the disease is cancer. In some embodiments, the cancer is breast cancer, ovarian cancer, endometrial cancer, bile duct cancer, gastric cancer, esophageal cancer, lung cancer, intestinal cancer, or pancreatic cancer. In yet some embodiments, the cancer is HER2+ breast cancer, triple negative breast cancer, or colorectal cancer.

In another aspect, the present application also discloses a method for preparing an antibody specifically binding to TRPV6, comprising: (1) coupling the amino acid sequence shown in SEQ ID NO: 3 to a carrier protein to obtain TRPV6 antigen peptide; (2) immunizing a mouse with the TRPV6 antigen peptide obtained by step (1) as an immunogen; (3) performing cell fusion, and screening and cloning of an immunopeptide to obtain a positive hybridoma cell line that efficiently secretes an antibody specifically binding to TRPV6; (4) obtaining the antibody specifically binding to TRPV6.

In some embodiments, the carrier protein is keyhole limpet hemocyanin (KLH) protein.

In some embodiments, a Cys is added to the N-terminus of the amino acid sequence shown in SEQ ID NO: 3, which is then coupled to the carrier protein via a free sulfhydryl group.
SEQ ID NO: 3: PLKPRTNNRTSPRDNTLLQQKLLQEAYMTPK.

In some embodiments, the hybridoma cell line of the present application is mouse hybridoma cell line 12CT 9.5.1, 16CT 18.1.1 or 16CT 4.1.1.2. The antibodies produced by cell line 12CT 9.5.1 include CB-Anti-0001 antibody, the antibodies produced by cell line 16CT4.1.1.2 include CB-Anti-0002 antibody, and the antibodies produced by cell line 16CT18.1.1 include CB-Anti- 0003 antibody.

In another aspect, the present application also discloses the use of the antibody or the antigen-binding fragment thereof of the present application, the multispecific antibody of the present application, the antibody conjugate of the present application and the pharmaceutical composition of the present application in a drug for treating and/or preventing and/or diagnosing diseases related to TRPV6.

In another aspect, the present application also discloses a method for treating, preventing or diagnosing diseases, wherein the method comprises administering the antibody or the antigen-binding fragment thereof of the present application, the multispecific antibody of the present application, the antibody conjugate of the present application and the pharmaceutical composition of the present application to a subject in need thereof.

### Examples

The following examples are intended to better illustrate the present invention, and should not be construed as a limitation to the scope of the present invention. All the following specific compositions, materials and methods, in whole or in part, are within the scope of the present invention. The particular compositions, materials and methods are not intended to limit the present invention but merely to illustrate that the particular embodiment is within the scope of the present invention. Those skilled in the art can develop equivalent compositions, materials and methods without adding inventive step or departing from the scope of the present invention. It should be understood that various modifications to the methods of the present invention can still be included within the scope of the present invention. The inventor intends to include such modifications in the scope of the present invention.

### Example 1 Synthesis of antigenic polypeptide and preparation of recombinant antigen

### 1. Antigen polypeptide selection

The sequence and secondary structure analysis was performed on the protein sequence (as shown in SEQ ID NO: 2) with the accession number of Q9H1D0 and GI: 1918017293 in Uniprot and Genbank. The full length of the TRPV6 protein is 765 amino acids, and the molecular weight thereof is about 87 kDa. The secondary structure and surface accessibility parameter of the protein were predicted by the online server http://www.cbs.dtu.dk/services/NetSurfP/, and the amino acid sequence IFQTEDPEELGHF (SEQ ID NO: 4), DGPANYNVDLPF (SEQ ID NO: 5) or PLKPRTNNRTSPRDNTLLQQKLLQEAYMTPK (SEQ ID NO: 3) was selected as the antigen and chemically synthesized according to the analysis results of the antigenic index thereof. To facilitate coupling, a cysteine was added to the amino end of the polypeptide to provide a sulfhydryl group.

### 2. Coupling and purification of polypeptide

Maleamide-activated keyhole limpet hemocyanin protein kit from Thermo Scientific was selected, and the step was performed following the procedure provided in the kit.

With regard to the polypeptide to be coupled, first, the free sulfhydryl group in the polypeptide was detected with Ellman reagent. 100 µL of stock solution of Ellman reagent was added to the 96-well plate, and then 10 µL of the polypeptide solution was added. The ultraviolet absorbance at λ = 412 nm of the mixture was detected with a Nano Drop spectrophotometer, and if the OD value > 0.15, the next step is performed; if the OD value is < 0.15 and > 0.05, the polypeptide is added until the requirement is met; and if the OD value is < 0.05, the peptide synthesis step is performed again for quality control.

When the coupling was started, 200 µL of deionized water was added to each mcKLH package to prepare a 10 mg/mL of KLH solution. 2 mg of hapten was dissolved in 500 µL of Imject EDC conjugation buffer, and 500 µL of polypeptide solution was added to 200 µL of carrier protein solution. l mL of deionized water was added to a packaged EDC (10 mg), and the mixture was shaken slowly until complete dissolution. 50 µL of the mixture was taken and added to the mcKLH polypeptide solution. The mixture was reacted for 2 hours, and then treated with desalting column to remove uncoupled cross-linking agent and salts.

### Example 2 Establishment of hybridoma cell line

### 1. Immunization

The cross-linked polypeptide in Example 1 was emulsified with complete Freund's adjuvant. 4-6 week-old female Balb/c mice (purchased from Beijing Vital River Laboratory Animal Technology Co., Ltd.) were immunized by subcutaneously injecting the mixture into the abdomen at 6 points of each mouse at a dose of 60 µg/mouse. The booster immunization was performed once every 14 days, the antigen was emulsified with incomplete Freund's adjuvant and the dose was 30 µg/mouse. 7 days after the third booster immunization, polyclonal antibody titer against the immunogen in the mouse serum was detected by indirect ELISA (at the wavelength of 450 nm). The mouse with the highest titer was subjected to rush immunization by tail vein injection, and the antigen was mixed uniformly with normal saline and the dose was 50 µg/mouse.

### 2. Cell fusion

The mouse splenocyte suspension that reaches the immune standard was prepared and mixed with the mouse myeloma cell F0 at a ratio of 5:1. The mixture was centrifuged at 1500 rpm for 5 minutes. After the supernatant was discarded, the centrifuge tube was put into a 37°C water bath. 1 mL of PEG1500 (Roche) was slowly added within 1 minute, and the cells were stirred. After the cells were allowed to stand in warm water for l minute, 10 mL of serum-free IMDM (Sigma) was added. The mixture was mixed uniformly, and centrifuged at 1000 rpm for 5 minutes. After the supernatant was discarded, 10 mL of serum (PAA) was added, and the cells wereblew carefully. 5 mL of thymocytes mixed with 10XHAT (Sigma) were added and mixed uniformly. Then 25 mL of semi-solid medium containing 2.1% nitrocellulose (Sigma) was added and mixed uniformly, and then the mixture was evenly poured into 20 cell culture dishes. The cell culture dishes were put into a humidifying box. The humidifying box was put into a 37°C, 5% CO₂ incubator and cultured.

### 3. Colonization and ELISA screening of positive hybridoma cell

7 days after fusion, the cloned cell clusters have moderate size and density. Round, solid and large cloned clusters were absorbed under an anatomic microscope, and put into a 96-well culture plate with the prepared medium. The culture plate was put into 37°C, 5% CO₂ incubator and cultured.

After 3 days, the cells accounted for approximately 2/3 of the bottom area. 100 µL of the supernatant was taken for ELISA screening with the immunogen and synthetic polypeptide, respectively. For positive clones, the medium was completely changed, and 200 µL of complete medium containing feeder cells and 1% HT (Sigma) was added. Two days later, the second ELISA screening was performed, and the positive clones were transferred to a 24-well plate with the prepared culture medium (containing feeder cells and HT) and cultured. Five days later, 100 µL of the supernatant was taken for the third ELISA screening, and three positive clones with high titers, 12CT 9.5.1, 16CT 18.1.1.2 and 16CT 4.1.1.2, were screened, successively transferred to a 6-well plate and cell culture flask to expand culture and cryopreserved.

### Example 3 Preparation of monoclonal antibody by ascites induction

### 1. Ascites preparation

Cells in logarithmic growth phase were washed with serum-free medium and suspended. The cells were counted and the concentration was about 5×10⁵/mL. l mL of suspended cells were intraperitoneally injected into the mouse presensitized with paraffin oil. Ascites collection was started 7 days later. The collected ascites was centrifuged at 4000 rpm for 10 minutes at 4°C. The ascites in the middle was carefully sucked out, collected in a centrifuge tube, and stored at 4°C or -20°C.

### 2. Purification of monoclonal antibody

The antibody was purified from the ascites by Protein G (GE) affinity chromatography according to the instruction. The purity was identified by SDS-PAGE gel, and the concentration was determined by Bradford method. The purified antibody was stored at -20°C.

### Example 4 Characteristics identification of monoclonal antibody

### 1. Subtype identification

Coated goat anti-mouse IgG was diluted to 0.5 µg/mL with 100 mL MPBS (pH 7.4), 100 µL of the mixture was added to each well, and the plate was incubated at 4°C overnight. The liquid was completely removed, and the residue was washed 3 times with PBS containing 0.05% Tween. 200 µL of blocking solution (PBS containing 2% BSA and 3% sucrose) was added to each well, and the mixture was incubated at 37°C for l hour. The liquid was completely removed, and the residue was washed 3 times with PBST. 0.l mL of hybridoma supernatant was added to each well, and the mixture was incubated at 37°C for l hour. The liquid was completely removed, and the residue was washed 3 times with PBST. HRP-labeled goat anti-mouse (κ, λ) antibody was diluted with the blocking solution at 1:1000 or HRP-labeled goat anti-mouse (IgM, IgG1, IgG2a, IgG2b, IgG3 and IgA) antibody was diluted with the blocking solution at 1:2000, which was added to appropriate wells at 0.l mL/well, and incubated at 37°C for l hour. The liquids were completely removed, and the residues were washed 3 times with PBST. 50 µL of citrate buffer containing 0.15% ABTS and 0.03% H₂O₂ (pH 4.0) was added to each well to perform colour reaction, and the OD value at a wavelength of 405 nm was measured within 10-20 minutes. The results showed that the obtained CB-Anti-0001 and CB-Anti-0002 antibodies were IgG1 type murine monoclonal antibodies, and the CB-Anti-0003 antibody was IgG2a type murine monoclonal antibody.

### 2. Determination of affinity constant

First, the obtained anti-TRPV6 antibody was immobilized on the SPR chip, then the immunopeptide was diluted to 11.52 nM with 1×PBST as the diluent, and then diluted 1.3-fold to the final concentration of 4.04 nM, and 5 concentration gradients in total were obtained. A SPR instrument was used for affinity detection, and the affinity constant of CB-Anti-0001 by automatic analysis using the instrument is calculated as 5.19×10⁻¹⁰.

### 3. Reaction specificity and use effect of monoclonal antibody

293T cells transfected with TRPV6 protein were selected, and the recognition specificity of the monoclonal antibody of the present invention was detected by an immunocytochemical method. The immunocytochemical experimental process was as follows. 293T cells were transfected with the TRPV6 plasmid, and the transfected cells were plated on a 24-well plate containing slides. The cells were cultured in IMDM+10% FBS medium for 72 hours, the supernatant was removed, and the residue was washed once with PBS, immobilized with methanol for 30 seconds, and washed twice with PBS. The anti-TRPV6 antibody working solution was prepared with an antibody diluent at an appropriate dilution ratio, PBS in the 24-well plate was removed, the antibody working solution was added, and the mixture was incubated overnight at 4 degrees Centigrade and washed with PBS three times. The secondary antibody (Elivision^{™} plus Polyer HRP (Mouse/Rabbit) IHC Kit, purchased from Fuzhou Maixin Biotechnology Co., Ltd., product number: KIT-9903) was added dropwise, and the mixture was incubated at room temperature for 15-20 minutes, and rinsed for 3X3 minutes with PBS (that is, the mixture was rinsed 3 times and each time lasted for 3 minutes). The PBS was discarded, and the residue was developed with freshly prepared DAB colour developing solution for 3 minutes. The sample was re-stained with hematoxylin for 25 seconds, and turned blue with PBS for 30 seconds. The sample was sequentially dehydrated with the gradients of 75%, 95%, 100% and 100% alcohol, finally cleared with xylene for 3 minutes, and sealed with neutral gum.

As shown in FIGS. 1A-1C, CB-Anti-0001 antibody, CB-Anti-0002 antibody and CB-Anti-0003 antibody can specifically detect TRPV6 protein by immunocytochemistry in 293T cells transfected with TRPV6 plasmid, respectively, and can not detect TRPV6 protein in 293T cells not transfected with TRPV6 plasmid. This result indicates that the obtained anti-TRPV6 antibody can specifically recognize TRPV6 protein, that is, the obtained anti-TRPV6 antibody has recognition characteristics.

### Example 5 Sequencing of antibody variable region

The cultured hybridoma cell line at 1×10⁶ was taken and centrifuged, and the supernatant was removed. The cell pellet was entrusted to GENEWIZ to determine the sequence of the antibody heavy chain and light chain variable regions, and exemplary antibody heavy chain variable region sequence and antibody light chain variable region sequence are shown below.

**Table 1 Amino acid sequence of antibody CDR**

| **Antibody** | | **CDR1** | **CDR2** | **CDR3** |
|---|---|---|---|---|
| **CB-Anti-0001** | HCDR | SEQ ID NO: 7 | SEQ ID NO: 8 | SEQ ID NO: 9 |
| | | GYTFTSYW | IDPSDGYT | GWAGDF |
| | LCDR | SEQ ID NO: 10 | SEQ ID NO: 11 | SEQ ID NO: 12 |
| | | QSLLDSDGKTY | LVS | WQGTHFPQT |
| **CB-Anti-0002** | HCDR | SEQ ID NO: 13 | SEQ ID NO: 14 | SEQ ID NO: 15 |
| | | GFTFSYYW | IRLKSHNYAT | ARLAYDGERYYYALDY |
| | LCDR | SEQ ID NO: 16 | SEQ ID NO: 17 | SEQ ID NO: 18 |
| | | QSLLYSNNQKNY | WAS | QQYYSYPFT |
| **CB-Anti-0003** | HCDR | SEQ ID NO: 19 | SEQ ID NO: 20 | SEQ ID NO: 21 |
| | | AFTFSHYW | IRLKSYNYAT | TRLGYFGSLYYAMDY |
| | LCDR | SEQ ID NO: 22 | SEQ ID NO: 23 | SEQ ID NO: 24 |
| | | QSLLYSTNQKNY | WAS | QQYYSYPWT |

**Table 2 Amino acid sequence of antibody variable region**

| **Antibody** (hybridoma cell strain) | **Heavy chain variable region** | **Light chain variable region** |
|---|---|---|
| **CB-Anti-0001 antibody** (12CT9.5.1) | SEQ ID NO: 25 | SEQ ID NO: 26 |
| | QVQVQQPGAEL VKPGTSVKLS CKASGYTFTSYWMHWVKQRP GQGLEWIGEIDPSDGYTNYNQ KFRGKATLSVDKSSTTAYMQL SSLTSEDSAVYYCGWAGDFWG QGTTLTVSS | DVVLTQSPLTLSVTIGQPASISC KSSQSLLDSDGKTYLNWLLQR PGQSPKRLIYLVSKLDSAAPDR FAGSGSGTDFTLKISRVEAEDL GIYYCWQGTHFPQTFGGGTKL EIK |
| **CB-Anti-0002** (16CT4.1.1.2) | SEQ ID NO: 27 | SEQ ID NO: 28 |
| | EVKLEESGGGLVQPGGSMKLS CVASGFTFSYYWMNWVRQSPE KGLEWVAEIRLKSHNYATHYA ESVKGRFTISRDDSKSSVYLQM NNLRSEDTGIYYCARLAYDGE RYYYALDYWGQGTSVTVSS | DIVMSQSPSSLAVSVGEKFTMS CKSSQSLLYSNNQKNYLAWY QQKPGQSPKLLIYWASTRESG VPDRFTGSGYGTDFTLTISSVK AEDLAVYYCQQYYSYPFTFGS GTKLEIK |
| **CB-Anti-0003** (16CT18.1.1) | SEQ ID NO: 29 | SEQ ID NO: 30 |
| | EVMLEESGGGLVQPGGSMKLS CVASAFTFSHYWMNWVRQSPE KGLEWVAEIRLKSYNYATHYA ESVKGRFTISRDDSKSSVYLQM NSLRPEDTGIYYCTRLGYFGSL YYAMDYWGQGTSVTVSS | DIVMSQSPSSLAVSVGEKVTM SCKSSQSLLYSTNQKNYLAWS QQKPGQSPKLLIYWASTESGVP DRFTGSGSGTDFTLTISSVKAE DLAVYYCQQYYSYPWTFGGG TKLEIK |

**Table 3 Nucleic acid sequence encoding antibody variable region**

| | **Heavy chain variable region** | **Light chain variable region** |
|---|---|---|
| **CB-Anti-0001 antibody** | SEQ ID NO: 31 | SEQ ID NO: 32 |
| | CAGGTCCAAGTGCAGCAGCCTGG GGCTGAGCTTGTGAAGCCTGGGA CTTCAGTGAAGCTGTCCTGCAAG GCTTCTGGCTACACCTTCACCAGC TACTGGATGCACTGGGTGAAGCA GAGGCCTGGACAAGGCCTTGAGT GGATCGGAGAGATTGATCCTTCT GATGGTTATACTAACTACAATCA AAAGTTCAGGGGCAAGGCCACAT TGTCTGTAGACAAATCCTCCACCA CAGCCTACATGCAGCTCAGCAGC CTGACATCTGAGGACTCTGCGGT CTATTACTGTGGGTGGGCGGGGG ACTTCTGGGGCCAAGGCACCACT CTCACAGTCTCCTCA | GATGTTGTGCTGACCCAGAGTCCAC TCACTTTGTCGGTTACCATTGGACA ACCAGCCTCCATCTCTTGCAAGTCA AGTCAGAGCCTCTTAGATAGTGATG GAAAGACATATTTGAATTGGTTGTT ACAGAGGCCAGGCCAGTCTCCAAAG CGCCTAATCTATCTGGTGTCTAAGCT GGACTCTGCAGCCCCTGACAGGTTC GCTGGCAGTGGATCAGGGACAGATT TCACACTGAAAATCAGCAGAGTGGA GGCTGAAGATTTGGGAATTTACTAT TGCTGGCAAGGTACACATTTTCCTC AGACGTTCGGTGGAGGCACCAAGCT GGAAATCAAA |
| **CB-Anti-0002** | SEQ ID NO: 33 | SEQ ID NO: 34 |
| | GAAGTGAAGCTTGAGGAGTCTGG AGGAGGCTTGGTGCAACCTGGAG GATCCATGAAACTCTCCTGTGTTG CCTCTGGATTCACTTTCAGTTACT ACTGGATGAACTGGGTCCGCCAG TCTCCAGAGAAGGGGCTTGAGTG GGTTGCTGAAATTAGATTGAAAT CTCATAATTATGCAACACATTATG CGGAGTCTGTGAAAGGGAGGTTC ACCATCTCAAGAGATGATTCCAA AAGTAGTGTCTACCTGCAAATGA ACAACTTAAGATCTGAAGACACT GGCATTTATTACTGTGCCAGGCTT | GACATTGTGATGTCACAGTCTCCAT CCTCCCTAGCTGTGTCAGTTGGAGA GAAGTTTACTATGAGCTGCAAGTCC AGTCAGAGCCTTTTATATAGTAACA ATCAAAAGAACTACTTGGCCTGGTA CCAGCAGAAACCAGGGCAGTCTCCT AAACTGCTGATTTACTGGGCATCCA CTAGGGAATCTGGGGTCCCTGATCG CTTCACAGGCAGTGGATATGGGACA GATTTCACTCTCACCATCAGCAGTG TGAAGGCTGAAGACCTGGCAGTTTA TTACTGTCAGCAATATTATAGCTATC |
| | GCTTACGACGGGGAGCGGTATTA CTATGCTCTGGACTACTGGGGTCA AGGAACCTCAGTCACCGTCTCCTC A | CATTCACGTTCGGCTCGGGGACAAA GTTGGAAATAAAA |
| **CB-Anti-0003** | SEQ ID NO: 35 | SEQ ID NO: 6 |
| | GAAGTGATGCTTGAGGAGTCTGG AGGAGGCTTAGTGCAACCTGGAG GATCCATGAAGCTCTCCTGTGTTG CCTCTGCATTCACTTTCAGTCACT ACTGGATGAACTGGGTCCGCCAG TCTCCAGAGAAGGGGCTTGAGTG GGTTGCTGAAATTAGATTGAAAT CTTATAATTATGCAACACATTATG CGGAGTCTGTGAAAGGGAGGTTC ACCATCTCCAGAGATGATTCCAA GAGTAGTGTCTACCTGCAAATGA ACAGCCTAAGGCCTGAAGACACT GGCATTTATTACTGTACCAGGCTT GGTTACTTCGGTAGCCTCTACTAT GCTATGGACTACTGGGGTCAAGG AACCTCAGTCACCGTCTCCTCA | GACATTGTGATGTCACAGTCTCCAT CCTCCCTAGCTGTGTCAGTTGGAGA GAAGGTTACTATGAGCTGCAAGTCC AGTCAGAGCCTTTTATATAGTACCA ATCAAAAGAACTACTTGGCCTGGTC CCAGCAGAAACCAGGGCAGTCTCCT AAACTGCTGATTTACTGGGCATCCA CTAGGGAATCTGGGGTCCCTGATCG CTTCACAGGCAGTGGATCTGGGACA GATTTCACTCTCACCATCAGCAGTG TGAAGGCTGAAGACCTGGCAGTTTA TTACTGTCAGCAATATTATAGCTATC CGTGGACGTTCGGTGGAGGCACCAA GCTGGAAATCAAA |

### Example 6 Tissue slice staining and identification

### 1. Slice preparation process

The tissue wax block was trimmed and serially sliced, and the thickness was 5 µm. The serial slices were unfolded in warm water at 45°C, and the slices were mounted with positron anti-off slides. The slides were air-dried at room temperature overnight, and then put at -20 °C for long-term storage.

### 2. IHC staining and analysis

The slides were routinely dewaxed twice with xylene for 15 minutes each time, hydrated in gradients of 100%, 100%, 95% and 75% ethanol for 3 minutes each time, and finally rinsed with tap water. Antigen retrieval was performed, then the slices were placed in a humidifying box and rinsed with PBS for 3X3 minutes. 3% H₂O₂ was added dropwise and the mixture was incubated for 10 minutes and rinsed with PBS for 3X3 minutes. The PBS was discarded, the blocking solution (animal non-immune serum) was added dropwise and the mixture was incubated at room temperature for 60 minutes. The slices were spin-dried, the primary antibody diluted in an appropriate ratio was added, and the mixture was incubated overnight at 4°C and rinsed with PBS for 3X3 minutes. The secondary antibody (Elivision^{™} plus Polyer HRP (Mouse/Rabbit) IHC Kit, purchased from Fuzhou Maixin Biotechnology Co., Ltd., product number: KIT-9903) was added dropwise, and the mixture was incubated at room temperature for 15-20 minutes, and rinsed for 3X3 minutes with PBS. The PBS was discarded, and the residue was developed with freshly prepared DAB colour developing solution for 3 minutes. The sample was re-stained with hematoxylin for 25 seconds, and turned blue with PBS for 30 seconds. The sample was sequentially dehydrated with the gradients of 75%, 95%, 100% and 100% alcohol, finally cleared with xylene for 3 minutes, and sealed with neutral gum.

### 3. Data statistics

As shown in FIGS. 2A-2C, CB-Anti-0001 antibody, CB-Anti-0002 antibody and CB-Anti-0003 antibody can be used to detect TRPV6 protein in the tissue slice by immunohistochemistry, respectively.

In addition, the inventor also used the H-score scoring method to record the staining of the tumour cell membrane. An H-score of more than 60 was recorded as strong staining, an H-score of 1-60 was recorded as weak staining, and the strong staining and weak staining were regarded as positive; and an H-score of 0 was recorded as negative. Statistics are compiled on the level of TRPV6 in each tumour tissue detected by IHC, and the level was compared with the TRPV6 mRNA level in the TCGA database and verified to evaluate the specificity of the antibody. The recorded information is listed in Table 4. The results show that when the antibody of the present invention is used to detect the TRPV6 protein level, the cancer types with high positive rate (especially high proportion of strong staining) correspondingly have high statistical TRPV6 mRNA level in the Cancer Genome Atlas Program (TCGA database) (e.g., TPM is greater than 1). It can be seen that the antibody of the present invention can be used to accurately assess the expression of TRPV6 on the cell surface, thereby determining whether the detected sample is a cancer cell expressing TRPV6.

**Table 4 Level of TRPV6 in tumour tissue detected by IHC**

| Tissue type | Number of samples | Strong staining | Weak staining | Positive | Negative | Expression level of TRPV6 mRNA in TCGA database (TPM, Transcript per million) |
|---|---|---|---|---|---|---|
| Bile duct cancer | 20 | 10% | 30% | 40% | 60% | 5 |
| Esophageal cancer | 20 | 0% | 0% | 0% | 100% | 0.35 |
| Gastric cancer | 20 | 0% | 10% | 10% | 90% | 0.2 |
| Her2⁺ breast cancer | 90 | 32% | 21% | 53% | 47% | 26 |
| Triple negative breast cancer | 100 | 48% | 26% | 74% | 26% | 9 |
| Ovarian cancer | 100 | 36% | 35% | 71% | 29% | 2 |
| Lung adenocarcinoma | 30 | 6% | 21% | 27% | 73% | 0.1 |

### Example 7 IHC comparative experiment

The commercially available antibody "Anti-Human TRPV6 (extracellular) Antibody" (Catalog No. ACC-028, purchased from Alomone Labs, RRID: AB_2756545, referred to as "ACC-028 antibody" for short), antibody "Anti-TrpV6 Antibody, clone 4A5.1" (Catalog No. MABN839, purchased from Sigma-Aldrich, referred to as "MABN839 antibody" for short) and CB-Anti-0001 antibody were used for comparative experiments.

The slice of the CDX tumour model RT4 (human bladder transitional cell papilloma cells) was obtained using the same procedure as in Example 6, section 2. ACC-028 antibody (the dilution ratio is 1:50), MABN839 antibody (the dilution ratio is 1:50) and CB-Anti-0001 antibody (the dilution ratio is 1:2000) were added dropwise, the mixtures were incubated overnight at 4°C and rinsed with PBS for 3X3 minutes. The secondary antibody (ElivisionTM plus Polyer HRP (Mouse/Rabbit) IHC Kit, purchased from Fuzhou Maixin Biotechnology Co., Ltd., product number: KIT-9903) was added dropwise, and the mixtures were incubated at room temperature for 15-20 minutes, and rinsed for 3X3 minutes with PBS. The PBS was discarded, and the residue was developed with freshly prepared DAB colour developing solution for 3 minutes. The sample was re-stained with hematoxylin for 25 seconds, and turned blue with PBS for 30 seconds. The sample was sequentially dehydrated with the gradients of 75%, 95%, 100% and 100% alcohol, finally cleared with xylene for 3 minutes, and sealed with neutral gum, and the staining results were read. The results showed that the detection sensitivity of the ACC-028 antibody was low, and the specific signals were only detected on the membranes of a small amount of RT4 cells (see FIG. 3A); the specificity of MABN839 antibody was poor, and the signals were detected in all tissue cells and interstitial cells (see FIG. 3B); and in contrast, for CB-Anti-0001 antibody, the positioning of detection signal is accurate and clear, and the specificity and sensitivity is high (see FIG. 3C), and the antibody is an ideal antibody for IHC applications.

### Example 8 Stability experiment

The stability of the CB-Anti-0001 antibody was verified by the accelerated destructive method. The method comprises (1) storing the antibody stock solution at 4°C for 14 days; (2) placing the antibody stock solution in a constant temperature incubator at 37°C for 14 days; and (3) placing the antibody working solution (obtained after diluting the antibody stock solution at a ratio of 1 :2000 with ZSbio ZLI-9028 antibody diluent) in a constant temperature incubator at 37°C for 14 days. Under the same experimental conditions, IHC detection was performed using the aforestated three antibody solutions to evaluate the stability of the antibodies treated with the three methods.

According to the detection results, the tissue staining intensities and staining ratios of the antibodies stored under different conditions did not change significantly (see FIG. 4, wherein FIG. 4A is the IHC result of the CB-Anti-0001 antibody stock solution stored at 4°C for 14 days, FIG. 4B is the IHC result of the CB-Anti-0001 antibody stock solution stored at 37°C for 14 days, and FIG. 4C is the IHC result of the CB-Anti-0001 antibody working solution stored at 37°C for 14 days). It can be seen that after the CB-Anti-0001 antibody working solution was stored at 37°C for 14 days, the antibody is still stable and retains good detection ability.

| | | |
|---|---|---|
| **0-1** | **Form PCT/RO/134 (SAFE) INDICATIONS RELATING TO DEPOSITED MICROORGANISM OR OTHER BIOLOGICAL MATERIAL (PCT Rule 13bis)** | |
| 0-1-1 | Software version | **CEPCT** |
| | | **Version 10. 25. 43 (20220101) MT/FOP** |
| | | **20140331/0. 20. 5. 21** |
| **0-2** | **International application number** | PCT/CN2022/071976 |
| **0-3** | **File Number of applicant or agent** | **0661388006W2** |
| | | |
| **1** | **The indications made below relate to the deposited microorganism or other biological material referred to in the description of the present application on page** | **paragraphs 28, 57 and 78** |
| **1-1** | | |
| **1-2** | **lines:** | |
| **1-3** | **Identification of deposit** | **China Center for Type Culture Collection Wuhan University, Wuhan City, Hubei Province, China, postal code: 430072, Hubei (CN). 13 January 2021 (13. 01. 2021) CCTCC C202119** |
| 1-3-1 | Name of depositary institution | |
| 1-3-2 | Address of depositary institution | |
| 1-3-3 | Date of deposit | |
| 1-3-4 | Accession number | |
| **1-4** | **Additional indications** | **Culture 1 name (classified nomenclature): Hybridoma cell strain 12CT 9.5.1** |
| **1-5** | **Designated states for which indications are made** | **All designated states** |
| **1-6** | **Separate furnishing of indications** | |
| | The indications listed below will be submitted to the International Bureau later | |
| **2** | **The indications made below relate to the deposited microorganism or other biological material referred to in the description of the present application on page** | **paragraphs 28, 57 and 78** |
| **2-1** | | |
| **2-2** | **lines:** | |
| **2-3** | **Identification of deposit** | **China Center for Type Culture Collection Wuhan University, Wuhan City, Hubei Province, China, postal code: 430072, Hubei (CN). 13 January 2021 (13. 01. 2021)** |
| 2-3-1 | Name of depositary institution | |
| 2-3-2 | Address of depositary institution | |
| 2-3-3 | Date of deposit | |
| 2-3-4 | Accession number | **CCTCC C202124** |
| **2-4** | Additional indications | **Culture 2 name (classified nomenclature): Hybridoma cell strain 16CT 4.1.1.2** |
| **2-5** | **Designated states for which indications are made** | **All designated states** |
| **2-6** | **Separate furnishing of indications** | |
| | The indications listed below will be submitted to the International Bureau later | |
| **3** | **The indications made below relate to the deposited microorganism or other biological material referred to in the description of the present application** | paragraphs 28, 57 and 78 |
| **3-1** | **on page** | |
| **3-2** | **lines:** | |
| **3-3** | **Identification of deposit** | **China Center for Type Culture Collection Wuhan University, Wuhan City, Hubei Province, China, postal code: 430072, Hubei (CN). 13 January 2021 (13. 01. 2021) CCTCC C202125** |
| 3-3-1 | Name of depositary institution | |
| 3-3-2 | Address of depositary institution | |
| 3-3-3 | Date of deposit | |
| 3-3-4 | Accession number | |
| **3-4** | **Additional indications** | **Culture 3 name (classified nomenclature): Hybridoma cell strain 16CT 18.1.1.2** |
| **3-5** | **Designated states for which indications are made** | **All designated states** |
| **3-6** | **Separate furnishing of indications** | |
| | The indications listed below will be submitted to the International Bureau later | |

### For receiving Office use only

| | | |
|---|---|---|
| **0-4** | **This sheet was received with the international application:** | |
| | (yes or no) | |
| 0-4-1 | Authorized officer | |

### For International Bureau use only

| | | |
|---|---|---|
| **0-5** | **This sheet was received by the International Bureau on:** | |
| 0-5-1 | Authorized officer | |

## Claims

1. An antibody or an antigen-binding fragment thereof specifically binding to TRPV6, wherein the antibody or the antigen-binding fragment thereof comprises three heavy chain complementarity determining regions HCDR1, HCDR2 and HCDR3 included in the following heavy chain variable region:
(a) a heavy chain variable region shown in SEQ ID NO: 25;
(b) a heavy chain variable region shown in SEQ ID NO: 27; or
(c) a heavy chain variable region shown in SEQ ID NO: 29.

2. The antibody or the antigen-binding fragment thereof according to claim 1, wherein the antibody or the antigen-binding fragment thereof comprises three heavy chain complementarity determining regions HCDR1, HCDR2 and HCDR3 selected from the group of
(a) HCDR1 shown in SEQ ID NO: 7, HCDR2 shown in SEQ ID NO: 8, and HCDR3 shown in SEQ ID NO: 9;
(b) HCDR1 shown in SEQ ID NO: 13, HCDR2 shown in SEQ ID NO: 14, and HCDR3 shown in SEQ ID NO: 15; or
(c) HCDR1 shown in SEQ ID NO: 19, HCDR2 shown in SEQ ID NO: 20, and HCDR3 shown in SEQ ID NO: 21.

3. The antibody or the antigen-binding fragment thereof according to claim 1, wherein the antibody or the antigen-binding fragment thereof further comprises three light chain complementarity determining regions LCDR1, LCDR2 and LCDR3 included in the following light chain variable region:
(a) a light chain variable region shown in SEQ ID NO: 26;
(b) a light chain variable region shown in SEQ ID NO: 28; or
(c) a light chain variable region shown in SEQ ID NO: 30.

4. The antibody or the antigen-binding fragment thereof according to claim 3, wherein the antibody or the antigen-binding fragment thereof further comprises three light chain complementarity determining regions LCDR1, LCDR2 and LCDR3 selected from the group of
(a) LCDR1 shown in SEQ ID NO: 10, LCDR2 shown in SEQ ID NO: 11 and LCDR3 shown in SEQ ID NO: 12;
(b) LCDR1 shown in SEQ ID NO: 16, LCDR2 shown in SEQ ID NO: 17 and LCDR3 shown in SEQ ID NO: 18; or
(c) LCDR1 shown in SEQ ID NO: 22, LCDR2 shown in SEQ ID NO: 23 and LCDR3 shown in SEQ ID NO: 24.

5. The antibody or the antigen-binding fragment thereof according to claim 1, wherein the antibody or the antigen-binding fragment thereof comprises three heavy chain complementarity determining regions HCDR1, HCDR2 and HCDR3 and three light chain complementarity determining regions LCDR1, LCDR2 and LCDR3 selected from the group of
(a) HCDR1 shown in SEQ ID NO: 7, HCDR2 shown in SEQ ID NO: 8, HCDR3 shown in SEQ ID NO: 9, LCDR1 shown in SEQ ID NO: 10, LCDR2 shown in SEQ ID NO: 11 and LCDR3 shown in SEQ ID NO: 12;
(b) HCDR1 shown in SEQ ID NO: 13, HCDR2 shown in SEQ ID NO: 14, HCDR3 shown in SEQ ID NO: 15, LCDR1 shown in SEQ ID NO: 16, LCDR2 shown in SEQ ID NO: 17 and LCDR3 shown in SEQ ID NO: 18; or
(c) HCDR1 shown in SEQ ID NO: 19, HCDR2 shown in SEQ ID NO: 20, HCDR3 shown in SEQ ID NO: 21, LCDR1 shown in SEQ ID NO: 22, LCDR2 shown in SEQ ID NO: 23 and LCDR3 shown in SEQ ID NO: 24.

6. The antibody or the antigen-binding fragment thereof according to claim 1, wherein the antibody or the antigen-binding fragment thereof comprises a heavy chain variable region V_{H} selected from the group of
(a) a heavy chain variable region shown in SEQ ID NO: 25;
(b) a heavy chain variable region shown in SEQ ID NO: 27; or
(c) a heavy chain variable region shown in SEQ ID NO: 29.

7. The antibody or the antigen-binding fragment thereof according to claim 6, wherein the antibody or the antigen-binding fragment thereof further comprises a light chain variable region V_{L} selected from the group of
(a) a light chain variable region shown in SEQ ID NO: 26;
(b) a light chain variable region shown in SEQ ID NO: 28; or
(c) a light chain variable region shown in SEQ ID NO: 30.

8. The antibody or the antigen-binding fragment thereof according to claim 1, wherein the antibody or the antigen-binding fragment thereof comprises a heavy chain variable region V_{H} and a light chain variable region V_{L} selected from the group of
(a) a heavy chain variable region shown in SEQ ID NO: 25 and a light chain variable region shown in SEQ ID NO: 26;
(b) a heavy chain variable region shown in SEQ ID NO: 27 and a light chain variable region shown in SEQ ID NO: 28; or
(c) a heavy chain variable region shown in SEQ ID NO: 29 and a light chain variable region shown in SEQ ID NO: 30.

9. The antibody or the antigen-binding fragment thereof according to any one of claims 1 to 8, wherein the antibody or the antigen-binding fragment thereof specifically recognizes or binds to a protein having an amino acid sequence shown in SEQ ID NO: 2.

10. The antibody or the antigen-binding fragment thereof according to claim 9, wherein the Kd value of the antibody or the antigen-binding fragment thereof to the protein having the amino acid sequence shown in SEQ ID NO: 2 is less than 10⁻⁷ M.

11. The antibody or the antigen-binding fragment thereof according to claim 10, wherein the Kd value is detected by a surface plasmon resonance method.

12. The antibody or the antigen-binding fragment thereof according to any one of claims 1 to 11, wherein the antigen-binding fragment is selected from F(ab')2, Fab', Fab, Fv, scFv, dsFv or dAb.

13. The antibody or the antigen-binding fragment thereof according to any one of claims 1 to 12, wherein the antibody or the antigen-binding fragment thereof further has a heavy chain constant region and/or a light chain constant region.

14. The antibody or the antigen-binding fragment thereof according to claim 13, wherein the heavy chain constant region is derived from murine IgG1 or murine IgG2a.

15. The antibody or the antigen-binding fragment thereof according to claim 13, wherein the heavy chain constant region is derived from human IgG1.

16. The antibody or the antigen-binding fragment thereof according to any one of claims 1 to 15, wherein the antibody or the antigen-binding fragment thereof further has a conjugated moiety.

17. The antibody or the antigen-binding fragment thereof according to claim 16, wherein the conjugated moiety is a therapeutic agent, a radioactive isotope, a detectable tag, a pharmacokinetically modified moiety or a purified moiety.

18. The antibody or the antigen-binding fragment thereof according to claim 15 or 16, wherein the conjugated moiety is linked directly or via a linker.

19. An isolated nucleic acid encoding the antibody or the antigen-binding fragment thereof according to any one of claims 1 to 18.

20. The nucleic acid according to claim 19, wherein the nucleic acid comprises:
(a) one or two of a nucleic acid sequence shown in SEQ ID NO: 31 or a nucleic acid sequence having at least 90% sequence identity to SEQ ID NO: 31, and a nucleic acid sequence shown in SEQ ID NO: 32 or a nucleic acid sequence having at least 90% sequence identity to SEQ ID NO: 32;
(b) one or two of a nucleic acid sequence shown in SEQ ID NO: 33 or a nucleic acid sequence having at least 90% sequence identity to SEQ ID NO: 33, and a nucleic acid sequence shown in SEQ ID NO: 34 or a nucleic acid sequence having at least 90% sequence identity to SEQ ID NO: 34; or
(c) one or two of a nucleic acid sequence shown in SEQ ID NO: 35 or a nucleic acid sequence having at least 90% sequence identity to SEQ ID NO: 35, and a nucleic acid sequence shown in SEQ ID NO: 6 or a nucleic acid sequence having at least 90% sequence identity to SEQ ID NO: 6.

21. A vector comprising the nucleic acid sequence according to claim 19 or 20.

22. A host cell comprising the vector according to claim 21.

23. A multispecific antibody comprising the antibody or the antigen-binding fragment thereof according to any one of claims 1 to 18, and one or more antibodies or antigen-binding portions thereof specifically binding to other antigens.

24. An antibody conjugate comprising the antibody or the antigen-binding fragment thereof according to any one of claims 1 to 18 or the multispecific antibody according to claim 23.

25. A hybridoma cell strain secreting an anti-TRPV6 monoclonal antibody, wherein the hybridoma cell strain is 12CT9.5.1, 16CT4.1.1.2 or 16CT18.1.1.2 and deposited in China Center for Type Culture Collection, and the deposit number of the 12CT9.5.1 is CCTCC NO: C202119, the deposit number of the 16CT4.1.1.2 is CCTCC NO: C202124, and the deposit number of the 16CT18.1.1.2 is CCTCC NO: C202125.

26. A pharmaceutical composition comprising the antibody or the antigen-binding fragment thereof according to any one of claims 1 to 18, the isolated nucleic acid according to any one of claims 19-20, the vector according to claim 21, the host cell according to claim 22, the multispecific antibody according to claim 23 or the antibody conjugate according to claim 24, and a pharmaceutically acceptable carrier.

27. A reagent for analyzing and detecting TRPV6 in a sample from a subject, wherein the reagent comprises one or more of the antibodies or antigen-binding fragments thereof according to any one of claims 1 to 18.

28. The reagent according to claim 27, wherein the reagent is used for analyzing and detecting TRPV6 in the sample from the subject.

29. A method for detecting TRPV6 in a sample from a subject, comprising:
(1) contacting the sample with the antibody or the antigen-binding fragment according to any one of claims 1-18;
(2) optionally, removing unbound antibodies or antibody fragments;
(3) detecting the antibody or antibody fragment binding to TRPV6 in the sample.

30. The method according to claim 29, wherein the antibody or the antigen-binding fragment according to any one of claims 1 to 18 in step (1) is immobilized on a substrate.

31. The method according to claim 29, wherein the sample in step (1) is immobilized on a substrate.

32. The method according to any one of claims 29 to 31, wherein the step (3) comprises using a second antibody, optionally, the second antibody can directly or indirectly bind to TRPV6 in the sample, optionally, the epitope of the second antibody is different from the epitope of the antibody or the antigen-binding fragment according to any one of claims 1 to 18, optionally, the epitope of the second antibody is the same as the epitope of the antibody or the antigen-binding fragment according to any one of claims 1 to 18, optionally, the binding of the epitope of the second antibody to TRPV6 is not affected by the antibody or antigen-binding fragment according to any one of claims 1 to 18, and optionally, the binding of the epitope of the second antibody to TRPV6 is affected by the antibody or antigen-binding fragment according to any one of claims 1 to 18.

33. The method according to any one of claims 29 to 31, wherein the step (3) comprises using a second antibody, optionally, the second antibody can directly or indirectly bind to the antibody or the antigen-binding fragment according to any one of claims 1 to 18, and optionally, the second antibody can bind to the constant region portion of the antibody or the antigen-binding fragment according to any one of claims 1 to 18.

34. The method according to claim 32 or 33, wherein the second antibody is coupled to a detection molecule, and optionally, the detection molecule includes an enzyme, a fluorescent label and biotin.

35. The method according to claim 34, wherein the detection molecule is an enzyme, and optionally, the enzyme includes horseradish peroxidase, alkaline phosphatase or a derivative thereof.

36. The method according to claim 34, wherein the detection molecule is fluorescein, optionally, the fluorescein includes FITC, rhodamine, Texas Red, phycoerythrin or Dylight.

37. The method according to claim 34, wherein the detection molecule is biotin or a derivative thereof.

38. The method according to any one of claims 29 to 37, wherein the method further comprises quantifying the antibody binding to TRPV6 in the sample.

39. The method according to claim 38, wherein the method is immunohistochemistry (IHC), enzyme-linked immunosorbent assay (ELISA), immunofluorescence (IF), chemiluminescent immunoassay (CLIA), turbidimetric immunoassay (TIA), or western blot.

40. The method according to any one of claims 29 to 39, wherein the subject is a human.

41. The method according to any one of claims 29 to 40, wherein the method is used to assess the proportion of the cells expressing TRPV6 on the cell surface in the sample.

42. A method for determining disease formation or the risk of forming a disease in a subject, assessing the progression or prognosis of a disease in a subject, or predicting or monitoring the response of a subject being receiving a treatment for a disease to the treatment in the subject, wherein the method comprises detecting TRPV6 in the sample from the subject using the method according to any one of claims 29 to 41.

43. The method according to claim 41, wherein the method comprises assessing the proportion of cells expressing TRPV6 on the cell surface in the sample, and if the proportion exceeds a predetermined threshold, the subject is considered to have the disease or be more likely to benefit from the treatment, and wherein the threshold is 10%, and preferably, the treatment is a targeted drug therapy.

44. The method according to claim 42 or claim 43, wherein the disease is cancer.

45. The method according to claim 44, wherein the cancer is breast cancer, ovarian cancer, endometrial cancer, bile duct cancer, gastric cancer, esophageal cancer, lung cancer, intestinal cancer, or pancreatic cancer; and preferably, the cancer is HER2⁺ breast cancer, triple negative breast cancer, or colorectal cancer.

46. A kit comprising the antibody or the antigen-binding fragment thereof according to any one of claims 1 to 18, and optionally, further comprising a reagent for detecting the binding of the antibody or the antigen-binding fragment thereof to TRPV6.

47. The kit according to claim 46, wherein the kit is used for diagnosing disease formation or the risk of forming a disease in a subject, assessing the progression or prognosis of a disease in a subject, or predicting or monitoring the response of a subject being receiving a treatment for a disease to the treatment in the subj ect.

48. The kit according to claim 46, wherein the kit is used for immunohistochemical pathological diagnosis.

49. The kit according to claim 48, wherein the kit is used for immunohistochemical pathological diagnosis of tumour tissue.

50. The kit according to claim 47, wherein the disease is cancer.

51. The kit according to claim 48, wherein the disease is breast cancer, ovarian cancer, endometrial cancer, bile duct cancer, gastric cancer, esophageal cancer, lung cancer, intestinal cancer, or pancreatic cancer; and preferably, the disease is HER2⁺ breast cancer, triple negative breast cancer, or colorectal cancer.

52. A method for preparing an antibody specifically binding to TRPV6, comprising:
(1) coupling the amino acid sequence shown in SEQ ID NO: 3 with a carrier protein to obtain TRPV6 antigen peptide;
(2) immunizing a mouse with the TRPV6 antigen peptide obtained by step (1) as an immunogen;
(3) performing cell fusion, and screening and cloning of an immunopeptide to obtain a positive hybridoma cell line that efficiently secretes an antibody specifically binding to TRPV6; and
(4) obtaining the antibody specifically binding to TRPV6.

53. The method according to claim 52, wherein the carrier protein is keyhole limpet hemocyanin (KLH) protein.

54. The method according to claim 52 or 53, wherein a Cys is added to the N-terminus of the amino acid sequence shown in SEQ ID NO: 3, which is then coupled to the carrier protein via a free sulfhydryl group.

55. The method according to any one of claims 52 to 54, wherein the hybridoma cell line is mouse hybridoma cell line 12CT 9.5.1, 16CT 18.1.1.2 or 16CT 4.1.1.2.

56. Use of the antibody or the antigen-binding fragment thereof according to any one of claims 1-18, the isolated nucleic acid according to any one of claims 19-20, the vector according to claim 21, the host cell according to claim 22, the multispecific antibody according to claim 23, the antibody conjugate according to claim 24 and the pharmaceutical composition according to claim 25 in a drug for treating and/or preventing and/or diagnosing diseases related to TRPV6.

57. A method for treating, preventing or diagnosing diseases, wherein the method comprises administering the antibody or the antigen-binding fragment thereof of any one of claims 1-18, the isolated nucleic acid according to any one of claims 19-20, the vector according to claim 21, the host cell according to claim 22, the multispecific antibody according to claim 23, the antibody conjugate according to claim 24 or the pharmaceutical composition according to claim 25 to a subject in need thereof.
